# FASCICULE DE BREVET EUROPEEN

(11) **EP 4 582 124 B1**
(45) Date de publication et mention de la délivrance du brevet: **13.05.2026**
(21) Numéro de dépôt: 24216379.8
(22) Date de dépôt: 29.11.2024
(51) Int. Cl.: A61M 16/00, A61M 16/10

(54) **DISPOSITIF DE DÉLIVRANCE DE NO AVEC SYSTÈME DE DOSAGE D'URGENCE**
NO-AUSGABEVORRICHTUNG MIT NOTFALLDOSIERSYSTEM
NO DELIVERY DEVICE WITH EMERGENCY DOSING SYSTEM

(30) Priorité: 03.01.2024 FR 2400028
(43) Date de publication de la demande: 09.07.2025
(73) Titulaire: INOSYSTEMS, 92160 Antony (FR)
(72) Inventeur: MARCHAL, Frédéric, 92182 Antony (FR); PATEL, Akshar, 92182 Antony (FR); BOULANGER, Thierry, 92182 Antony (FR)
(74) Mandataire: Air Liquide

(56) Documents cités:
- WO-A1-97/31670
- FR-A1- 2 911 281
- FR-A1- 3 131 538
- US-A1- 2014 261 415
- US-A1- 2015 233 879
- US-B2- 10 894 135
- US-B2- 9 351 994

## Description

L'invention concerne un dispositif de délivrance de monoxyde d'azote gazeux (NO) à un patient comprenant un système de dosage d'urgence de NO, et destiné à être connecté au circuit patient d'un ventilateur mécanique, c'est-à-dire un appareil médical d'administration de gaz à un patient, lequel permet de fournir le gaz à débit préfixé en cas de dysfonctionnement, notamment des moyens de pilotage.

Le NO est un gaz qui, lorsqu'inhalé, dilate les vaisseaux pulmonaires et augmente l'oxygénation en améliorant les échanges gazeux. Il est utilisé pour traiter différentes conditions médicales, comme l'Hypertension Artérielle Pulmonaire du nouveau-né ou PPHN (pour *Persistent Pulmonary Hypertension of the Newborn*), le Syndrome de Détresse Respiratoire Aigüe ou SDRA observé principalement chez l'adulte ou encore les hypertensions pulmonaires en chirurgie cardiaque, comme enseigné notamment par EP-A-560928, EP-A-1516639 ou US-A-10,201,564.

Usuellement, une faible quantité de NO gazeux (i.e. quelques ppm vol.), dilué dans de l'azote (N₂) est injecté dans un flux gazeux contenant de l'oxygène (O₂) qui est ensuite inhalé par le patient. La concentration de NO, qui correspond à une posologie, est déterminée par le médecin ou analogue. Typiquement, le gaz contenant l'O₂ est typiquement un mélange N₂/O₂ ou de l'air, tel de l'air de qualité médical. En général, la concentration de NO dans le gaz inhalé par le patient est comprise entre 1 et 80 ppm en volume (ppmv), en fonction de la population traitée, i.e. nouveau-nés ou adultes, et donc de la maladie à traiter.

Le gaz inhalé par le patient peut être délivré par le biais d'un dispositif de délivrance de NO associé à un ventilateur mécanique, comme décrit par US-A-5,558,083. Le dispositif de délivrance de NO est fluidiquement connecté à une ou plusieurs bouteilles de gaz contenant un mélange de N₂/NO dont la concentration en NO peut être comprise typiquement entre 200 et 1000 ppmv. Généralement, le système de délivrance de NO comprend un module d'injection de NO placé dans la branche inspiratoire d'un circuit patient connecté fluidiquement, d'une part, au ventilateur mécanique et, d'autre part, à une interface respiratoire délivrant le gaz enrichi en NO au patient, par exemple un masque respiratoire, une sonde d'intubation trachéale ou similaire.

Le système de délivrance de NO comprend également un capteur de débit qui mesure le débit gazeux délivré par le ventilateur mécanique (i.e. air ou mélange N₂/O₂) afin de déterminer la quantité de NO à délivrer pour respecter la posologie fixée par le médecin.

Le système de délivrance de NO peut assurer le dosage en NO par le biais d'une électrovanne proportionnelle délivrant un flux continu de gaz contenant le NO, laquelle est associée à un capteur de débit, les deux composants étant agencés dans le système de délivrance, ainsi qu'une ligne d'injection reliée au module d'injection de NO, tel que décrit dans US-A-5,558,083.

Un autre système de délivrance de NO est connu, par exemple, du document FR 3 131 538 A1.

D'autres systèmes sont disponibles où l'électrovanne proportionnelle est remplacée par une pluralité d'électrovannes de type « tout ou rien », délivrant le gaz de façon intermittente, c'est-à-dire sous forme de pulses, généralement à haute fréquence, dont l'amplitude et la durée permettent de garantir la bonne quantité de gaz circulant dans la ligne d'injection reliée au module d'injection de NO.

Dans tous les cas, les systèmes de délivrance de NO connus reçoivent les mesures du capteur de débit placé dans la branche inspiratoire du circuit patient et ajustent en temps réel la quantité de NO devant être délivrée, selon la posologie désirée, en contrôlant le flux de NO dans la ligne d'injection.

Le NO étant un agent thérapeutique efficace, c'est-à-dire que de très faibles concentrations (i.e. quelques ppmv) produisent un effet thérapeutique, son juste dosage est d'importance critique et les équipes médicales doivent constamment adapter la posologie selon l'état du patient.

Lorsque l'état du patient évolue, la concentration de NO doit être progressivement diminuée ou augmentée. Par exemple, dans une situation de sevrage du nouveau-né dont l'état s'améliore, il est usuel de décroitre progressivement la posologie, par exemple par pas de 1 ppm, jusqu'à atteindre une valeur nulle permettant alors de stopper le système de délivrance de NO.

Une diminution progressive de la concentration en NO permet d'éviter l'effet « rebond » pouvant se manifester en cas de variation rapide de la concentration, par exemple en cas de discontinuation brutale du traitement, avec pour effet d'empirer gravement l'état du patient.

Or, les appareils de délivrance de NO sont des systèmes électro-médicaux sophistiqués susceptibles de subir des défaillances ou dysfonctionnements pouvant avoir un impact important sur la thérapie en cours. Par exemple, un dysfonctionnement ou défaut électronique majeur, en particulier des moyens de pilotage, peut entrainer une panne de l'appareil et donc un arrêt total de délivrance de NO, avec les conséquences négatives susmentionnées.

Dans de telles circonstances, l'appareil de délivrance de NO doit avertir l'utilisateur, par exemple au moyen d'un signal d'alarme audible, qu'une action rapide est requise, typiquement un basculement vers un mode d'injection pneumatique de secours, i.e. un mode dit « de secours », afin de limiter autant que possible, les effets indésirables liés à une discontinuation de la thérapie.

Un tel basculement en mode de secours se fait habituellement par actionnement d'un organe de commande, tel un bouton rotatif, commandant par exemple une délivrance continue d'un débit fixe de NO, typiquement de mélange N₂/NO, par exemple de l'ordre de 250 mL/min.

Or, un mécanisme ou système de dosage de secours n'est pas sans risque, notamment pour les raisons suivantes :
- son activation requiert la présence d'une personne ayant autorité pour entreprendre cette action, par exemple un médecin en néonatologie. En milieu hospitalier, il peut se passer plusieurs minutes avant que cette personne n'arrive dans la salle de soins et donc que le dosage de secours ne soit établi, ce qui peut entrainer un arrêt momentané de la thérapie et exposer le patient à un effet rebond.
- le dosage de secours, tel un débit préfixé de mélange N₂/NO, ne permet pas de garantir que la posologie désirée soit toujours respectée. En particulier, quand le dosage de secours est très inférieur à la posologie souhaitée, le patient peut être exposé à un changement abrupte de concentration et potentiellement sujet à d'importants effets indésirables, ce qui n'est pas souhaitable pour d'évidentes raisons de sécurité et d'efficacité du traitement du patient.
- le dosage de secours est incompatible avec certains types de ventilateurs délivrant de très faibles volumes, tels que les ventilateurs de type HFO à oscillations à haute fréquence (*High Frequency Oscillations*) car il peut en résulter une concentration en NO inhalée trop élevée pouvant même parfois atteindre des niveaux dangereux pour le patient. Dès lors, en cas de traitement du patient avec un ventilateur HFO, on se retrouve alors sans moyens d'administrer le NO au patient, ce qui entraine les risques susmentionnés liés à l'arrêt brusque de traitement.

Il apparaît dès lors que les mécanismes de dosage de secours actuels ne permettant pas de garantir un niveau satisfaisant de sécurité et qu'il serait souhaitable pour le patient, en cas de mise en place d'un dosage de secours du fait d'un dysfonctionnement de l'appareil de délivrance de NO, de pouvoir maintenir une thérapie par NO, sans commettre d'interruption de la thérapie et/ou sans se soucier du type de ventilateur, i.e. ventilateur HFO ou autre, avec lequel coopère l'appareil de délivrance de NO.

Autrement dit, un problème est de pouvoir maintenir une posologie, c'est-à-dire un traitement du patient par NO inhalé, même en cas de défaillance ou dysfonctionnement de l'appareil de délivrance de NO, en particulier un arrêt total de fonctionnement des moyens de pilotage de l'appareil de délivrance de NO, en particulier du fait d'une panne, d'un dysfonctionnement ou d'un défaut d'alimentation électrique.

Une solution selon l'invention concerne un dispositif ou appareil de délivrance de NO pour fournir un gaz contenant du NO, typiquement un mélange gazeux NO/azote, comprenant :
- une ligne d'injection de NO pour acheminer le gaz contenant du NO,
- un dispositif à vanne agencé sur la ligne d'injection pour contrôler la circulation du gaz contenant du NO dans la ligne d'injection, ledit dispositif à vanne étant configuré pour être normalement dans une position fermée pour empêcher toute circulation de gaz dans la ligne d'injection,
- un dispositif de mesure de débit agencé sur la ligne d'injection pour opérer une ou des mesures de débit du gaz contenant du NO circulant dans la ligne d'injection,
- un circuit de secours comprenant une ligne de secours venant se raccorder fluidiquement à la ligne d'injection, en amont et en aval du dispositif à vanne, ladite ligne de secours comprenant une électrovanne de secours configurée pour être normalement dans une position ouverte pour permettre une circulation de gaz dans la ligne de secours, et un dispositif de contrôle de débit, et
- des moyens de pilotage, i.e. une unité de pilotage, configurés pour coopérer avec l'électrovanne de secours, le dispositif de contrôle de débit, le dispositif à vanne et le dispositif de mesure de débit.

En cas de dysfonctionnement engendrant un arrêt de coopération avec les moyens de pilotage, c'est-à-dire en cas de dysfonctionnement des moyens de pilotage par exemple du fait d'un défaut d'alimentation électrique :
- l'électrovanne de secours est configurée pour passer en position ouverte pour permettre une circulation de gaz dans la ligne de secours du circuit de secours,
- le dispositif à vanne est configuré pour passer en position fermée pour stopper toute circulation de gaz dans la ligne d'injection, et
- le dispositif de contrôle de débit est configuré pour fournir le gaz à un débit gazeux de secours préfixé, où ledit débit gazeux de secours :
   ∘ est déterminé par les moyens de pilotage à partir d'au moins une mesure de débit du gaz fournie par le dispositif de mesure de débit, pendant un fonctionnement normal de l'appareil précédant ledit dysfonctionnement, et
   ∘ est préréglé par commande dudit dispositif de contrôle de débit par les moyens de pilotage, pendant ledit fonctionnement normal de l'appareil.

De plus :
- une électrovanne à plusieurs voies est agencée sur la ligne de secours, en aval du dispositif de contrôle de débit,
- ladite électrovanne à plusieurs voies comprenant :
   ∘ une voie d'entrée raccordée fluidiquement à la ligne de secours en aval du dispositif de contrôle de débit,
   ∘ une première voie de sortie raccordée fluidiquement à une première ligne de dosage comprenant un premier dispositif à orifice calibré, et
   ∘ une seconde voie de sortie raccordée fluidiquement à une seconde ligne de dosage comprenant un second dispositif à orifice calibré,
- la première ligne de dosage et la seconde ligne de dosage viennent se raccorder à la ligne de secours, en aval desdits premier et second dispositif à orifice calibré, et
- les premier et second dispositifs à orifice calibré présentent des orifices calibrés de sections de passage ou diamètres différents,
- les moyens de pilotage sont configurés pour piloter l'électrovanne à plusieurs voies pour diriger le flux gazeux vers la première ligne de dosage ou, alternativement, vers la seconde ligne de dosage en fonction de la ou des mesures de débit de gaz fournies par le dispositif de mesure de débit lors du fonctionnement normal du dispositif précédant ledit dysfonctionnement.

Selon le mode de réalisation considéré, l'appareil de l'invention peut comprendre l'une ou plusieurs des caractéristiques suivantes :
- l'électrovanne à plusieurs voies comprend 3 voies.
- la voie d'entrée de l'électrovanne à plusieurs voies est alimentée en gaz par la ligne de secours, typiquement en mélange NO/N₂, en cas de dysfonctionnement de l'appareil de délivrance de NO, en particulier de l'unité de pilotage, typiquement en défaut de défaut d'alimentation électrique de ladite unité de pilotage.
- le dysfonctionnement engendrant un arrêt de (toute) coopération avec les moyens de pilotage comprend un défaut desdits moyens de pilotage ou un défaut d'alimentation électrique desdits moyens de pilotage.
- en cas de dysfonctionnement de l'appareil de délivrance de NO, la première voie de sortie de l'électrovanne à plusieurs voies alimente la première ligne de dosage comprenant le premier dispositif à orifice calibré.
- alternativement, en cas de dysfonctionnement de l'appareil de délivrance de NO, la seconde voie de sortie de l'électrovanne à plusieurs voies alimente la seconde ligne de dosage comprenant le second dispositif orifice calibré.
- la voie d'entrée de gaz de l'électrovanne à plusieurs voies comprend un port amont recevant le gaz, typiquement un mélange gazeux NO/N₂,
- la première voie de sortie de l'électrovanne à plusieurs voies comprend un premier port aval fournissant le gaz, typiquement un mélange gazeux NO/N₂, à la première ligne de dosage.
- la seconde voie de sortie de l'électrovanne à plusieurs voies comprend un second port aval fournissant le gaz, typiquement un mélange gazeux NO/N₂, à la seconde ligne de dosage.
- les moyens de pilotage sont configurés pour, pendant le fonctionnement normal de l'appareil, commander l'électrovanne de secours pour qu'elle soit en une position fermée empêchant toute circulation de gaz dans la ligne de secours.
- les moyens de pilotage sont configurés pour, pendant le fonctionnement normal de l'appareil, commander le dispositif à vanne pour autoriser une circulation de gaz dans la ligne d'injection et de préférence permettre d'opérer au moins une mesure de débit de gaz par le dispositif de mesure de débit.
- les moyens de pilotage sont en outre configurés pour, pendant le fonctionnement normal de l'appareil, contrôler le dispositif de contrôle de débit pour prérégler le débit gazeux de secours à partir d'au moins une mesure de débit du gaz fournie par le dispositif de mesure de débit. Autrement dit, le réglage du dispositif de contrôle de débit se fait préalablement à tout dysfonctionnement, c'est-à-dire pendant que de l'appareil de fourniture de NO fonctionne normalement.
- pendant le fonctionnement normal de l'appareil, le dispositif de mesure de débit est configuré pour opérer plusieurs mesures de débit successives.
- pendant le fonctionnement normal de l'appareil, les moyens de pilotage sont en outre configurés pour déterminer, par exemple calculer, le débit gazeux de secours (i.e. débit de gaz contenant du NO, e.g. mélange NO/N₂) à partir d'une ou plusieurs mesures de débit opérées par le dispositif de mesure de débit.
- la ligne de secours vient se raccorder fluidiquement à la ligne d'injection en amont du dispositif à vanne, et en amont ou en aval du dispositif de mesure de débit, de préférence en aval du dispositif de mesure de débit.
- un dispositif de mesure de débit est agencé sur la ligne d'injection en amont ou en aval du dispositif à vanne, de préférence en aval du dispositif à vanne.
- la ligne de secours vient se raccorder fluidiquement à la ligne d'injection par une extrémité amont, en amont du dispositif à vanne et par une extrémité aval, en aval du dispositif à vanne de manière à bipasser ledit dispositif à vanne.
- la ligne de secours vient se raccorder fluidiquement à une portion amont de la ligne d'injection située en amont du dispositif à vanne, en particulier via son extrémité amont.
- la ligne de secours vient se raccorder fluidiquement à une portion aval de la ligne d'injection située en aval du dispositif à vanne, en particulier via son extrémité aval.
- il comprend des moyens de mémorisation pour mémoriser au moins une partie des mesures de débit successives opérées par le dispositif de mesure de débit, c'est-à-dire les mesures de débit successives sont mémorisées par des moyens de mémorisation.
- les moyens de mémorisation sont configurés pour mémoriser par ailleurs une ou des tables de correspondance.
- les moyens de mémorisation sont configurés pour mémoriser par ailleurs au moins une table de correspondance donnant une relation entre pression et débit du ou des dispositif à orifice calibrés.
- les moyens de mémorisation comprennent une mémoire informatique, par exemple un mémoire vive, ou autre.
- la ligne d'injection de NO achemine un mélange gazeux formé de NO et d'azote, de préférence un mélange gazeux NO/N₂ (i.e. monoxyde d'azote/azote) contenant entre 100 et 2000 ppmv de NO, typiquement moins de 1000 ppmv de NO, le reste étant de l'azote (et éventuellement des impuretés inévitables)
- l'électrovanne de secours est configurée pour être normalement ouverte, en particulier lorsqu'elle n'est pas pilotée par les moyens de pilotage, typiquement en cas de dysfonctionnement.
- l'électrovanne de secours est du type tout ou rien.
- les moyens de pilotage comprennent au moins un microprocesseur.
- les moyens de pilotage comprennent une carte électronique portant ledit au moins un microprocesseur.
- la ligne d'injection est raccordée fluidiquement à une ligne haute pression par l'intermédiaire d'un dispositif régulateur de pression, la ligne haute pression et le dispositif régulateur de pression étant agencés dans l'appareil de délivrance de NO.
- le dispositif de délivrance de NO comprend un boitier.
- le système de dosage de secours de NO est agencé dans le boitier, en particulier la ligne de secours et l'électrovanne de secours.
- la ligne de secours vient se raccorder fluidiquement à la ligne d'injection entre le dispositif régulateur de pression et le dispositif à vanne.
- le dispositif à vanne comprend une électrovanne, de préférence une électrovanne proportionnelle.
- le dispositif de contrôle de débit est configuré pour former ou constituer un système de génération de pression et de débit proportionnel
- le dispositif de contrôle de débit comprend un moyen actionneur coopérant avec un régulateur de pression pneumatique.
- le dispositif de contrôle de débit comprend un moyen actionneur permettant de contrôler le niveau de pression de sortie du régulateur de pression pneumatique.
- le dispositif de contrôle de débit comprend un moyen actionneur à réglage par déplacement angulaire.
- le moyen actionneur comprend un moteur électrique, en particulier un moteur pas à pas.
- le moyen actionneur est alimenté par les moyens d'alimentation électrique, i.e. pendant le fonctionnement normal.
- le moyen actionneur comprend un moteur électrique entrainant un axe rotatif, solidaire du régulateur de pression pneumatique.
- le régulateur de pression pneumatique comprend un port d'entrée et un port de sortie en communication fluidique avec la ligne de secours.
- les moyens de pilotage sont configurés pour piloter le moyen actionneur pour opérer un déplacement, de préférence angulaire, du régulateur de pression pneumatique entre au moins:
   ∘ une position d'ouverture totale correspondant à un niveau d'ouverture maximale, c'est-à-dire correspondant à une pression maximale (et de débit maximal) du régulateur de pression pneumatique. En position d'ouverture totale, tout le débit gazeux amené par la ligne de secours pénètre dans le régulateur de pression pneumatique, c'est-à-dire un débit maximal,
   ∘ une position de fermeture totale correspondant à un niveau de fermeture totale, c'est-à-dire correspondant à une pression nulle (et à débit nul), du régulateur de pression pneumatique. En position de fermeture totale, aucun débit gazeux ne peut traverser le régulateur de pression, c'est-à-dire un débit nul,
   ∘ et avantageusement au moins une position intermédiaire située entre lesdites positions d'ouverture maximale et de fermeture maximale, donc correspondant à un niveau de pression en sortie du régulateur de pression pneumatique comprise entre la valeur de pression maximale et la valeur de pression nulle (i.e. 0 bar). En position intermédiaire, seulement une partie du débit gazeux amené par la ligne de secours pénètre dans le régulateur de pression pneumatique, c'est-à-dire un ou des débits réduits ou limités et inférieurs au débit maximal.
- les moyens de pilotage sont configurés pour piloter le moyen actionneur pour opérer un déplacement angulaire du régulateur de pression pneumatique entre plusieurs positions angulairement distinctes, angulairement décalées les uns des autres, comprenant la position d'ouverture totale, la position de fermeture totale et plusieurs positions intermédiaires situées entre les positions d'ouverture totale et de fermeture totale. Lesdites positions angulairement distinctes correspondent chacune à un niveau de pression de sortie et à un débit de gaz donné, c'est-à-dire des débits compris entre le débit maximal, le débit nul et des débits intermédiaires compris entre ces débits maximal et nul.
- les moyens de pilotage sont configurés pour piloter, commander ou contrôler le moyen actionneur, pendant le fonctionnement normal du dispositif, c'est-à-dire préalablement à tout dysfonctionnement, de manière à régler ou ajuster le débit gazeux de secours préfixé, i.e. le débit souhaité.
- les moyens de pilotage sont configurés pour piloter le moyen actionneur pour opérer un déplacement, de préférence angulaire, d'un élément mobile dudit moyen actionneur dans une position donnée correspondant au débit de secours préréglé.
- l'élément mobile comprend un arbre rotatif, de préférence il est en métal ou alliage métallique.
- l'élément mobile comprend un arbre rotatif apte à être entraîner en rotation par un moteur électrique.
- les moyens de pilotage sont configurés pour déterminer l'ouverture du régulateur de pression pneumatique et/ou le débit de secours préréglé à partir d'une table de correspondance mémorisée.
- les moyens de pilotage sont configurés pour déterminer une ouverture du régulateur de pression pneumatique correspondant au débit de secours préréglé.
- la table de correspondance est mémorisée par les moyens de mémorisation, telle une mémoire informatique.
- il comprend des moyens d'alimentation électrique configurés pour alimenter en courant électrique les composants nécessitant de l'énergie électrique pour fonctionner, notamment les moyens de pilotage ou d'autres composants, comme les électrovannes, le moteur électrique....
- les moyens d'alimentation électrique comprend des moyens de raccordement au secteur (110/220V) et/ou une batterie ou analogue.
- le dispositif de contrôle de débit du système de dosage de secours de NO, qui forme un système proportionnel, permet de (pré)régler ou ajuster le débit gazeux de secours préfixé, préalablement à tout dysfonctionnement de l'appareil empêchant toute coopération entre les moyens de pilotage et l'électrovanne de secours, le dispositif de contrôle de débit, le dispositif à vanne et/ou le dispositif de mesure de débit.
- le débit gazeux de secours mesuré par un capteur de débit de NO situe dans la ligne d'injection de NO correspond à la dernière mesure de débit opérée par le dispositif de mesure de débit de NO ayant été opérée avant le dysfonctionnement.
- le premier orifice calibré du premier dispositif à orifice calibré présente un premier diamètre de passage (D1) et le second orifice calibré du second dispositif à orifice calibré présente un second diamètre de passage (D2) tels que 1,5.D1 < D2 < 4.D1.
- le premier diamètre de passage (D1) et le second diamètre de passage (D2) sont tels que 1,8.D1 < D2 < 3.D1, de préférence D2 est égal à environ 2.D1.
- l'électrovanne de secours est du type tout ou rien pouvant adopter uniquement un état ouvert dans lequel elle laisse passer le flux gazeux et un état fermé dans lequel elle interrompt le passage de flux gazeux.
- le dispositif de contrôle de débit comprenant un moyen actionneur coopérant avec un régulateur de pression pneumatique.
- le moyen actionneur comprend un moteur pas à pas, de préférence un moteur électrique.
- le régulateur pneumatique est configuré pour être réglable sur plusieurs niveaux de pression compris entre 0 et 2 bar relatif, de préférence moins de 1,5 bar relatif.
- le moyen actionneur coopère avec le régulateur pneumatique pour fixer une pression de sortie souhaitée en aval dudit régulateur pneumatique.
- le régulateur pneumatique comprend un ressort interne permettant de régler le niveau de pression souhaité.
- le moyen actionneur comprend un moteur pas à pas configuré pour adopter plusieurs positions angulaires différentes, chaque position angulaire du moteur pas-à-pas correspondant à une tension donnée du ressort interne du régulateur pneumatique.
- le niveau de pression de sortie souhaité en aval du régulateur pneumatique est déterminé par la tension du ressort interne du régulateur pneumatique correspondant à la position angulaire adoptée par le moteur pas-à-pas.
- en fonctionnement normal, les moyens de pilotage sont configurés pour piloter l'électrovanne à plusieurs voies pour opérer une communication fluidique entre la voie d'entrée de l'électrovanne à plusieurs voies et l'une ou l'autre des première et seconde voies de sortie de l'électrovanne à plusieurs voies de manière à faire passer le flux gazeux au travers du premier ou du deuxième dispositif à orifice calibré.

L'invention concerne aussi une installation de fourniture de gaz à un patient, c'est-à-dire un être humain, comprenant :
- au moins une source de NO contenant un mélange gazeux NO/N₂,
- un dispositif de délivrance de NO selon l'invention, alimenté en mélange gazeux NO/N₂ par ladite au moins une source de NO,
- une branche inspiratoire d'un circuit patient alimentée en mélange gazeux NO/N₂ par le dispositif de délivrance de NO, et
- un ventilateur médical, i.e. un appareil d'assistance respiratoire, en communication fluidique avec la branche inspiratoire pour alimenter ladite branche inspiratoire en un gaz respiratoire contenant au moins 20% d'oxygène.

Selon le mode de réalisation considéré, l'installation de fourniture de gaz de l'invention peut comprendre l'une ou plusieurs des caractéristiques suivantes :
- le ventilateur médical délivre de l'air ou un mélange oxygène/azote, i.e. en tant que gaz respiratoire contenant au moins 21% vol. d'oxygène.
- selon un mode de réalisation, le ventilateur médical comprend une soufflante motorisée (i.e. turbine, compresseur ou analogue) délivrant le gaz respiratoire, typiquement de l'air ou un mélange oxygène/azote.
- selon un autre mode de réalisation, le ventilateur médical comprend un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens de commande, telle une (ou des) carte de commande électronique.
- les moyens de commande, telle une carte de commande électronique, pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- la source de NO contient un mélange gazeux NO/N₂ contenant entre 100 et 2000 ppmv de NO, le reste étant de l'azote (N₂), conditionné à une pression comprise entre 10 et 250 bar abs, typiquement à plus de 100 bar abs (avant début de soutirage).
- la source de NO contient un mélange gazeux NO/N₂ contenant entre 100 et 1000 ppmv de NO, le reste étant de l'azote (N₂), conditionné à une pression comprise entre 10 et 250 bar abs, typiquement à plus de 100 bar abs (avant début de soutirage).
- la source de NO est une (ou des) bouteille de gaz sous pression.
- la source de NO est une (ou des) bouteille de gaz ayant une contenance comprise entre 0,5 et 50 L (équivalent en eau).
- la bouteille de gaz comprend un corps cylindrique en acier ou en alliage d'aluminium.
- la bouteille de gaz est équipée d'un robinet simple (sans détendeur) ou à détendeur intégré ou RDI.
- la bouteille de gaz est équipée d'un RDI protégé par un capotage de protection, par exemple en métal ou polymère.
- le circuit patient comprend une branche inspiratoire et une branche expiratoire.
- le circuit patient comprend des conduites flexibles formant la branche inspiratoire et la branche expiratoire, typiquement des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire.
- la branche inspiratoire et la branche expiratoire comprennent des conduites flexibles, par exemple en polymère.
- la branche inspiratoire et la branche expiratoire sont en outre fluidiquement raccordées à, respectivement, des orifices de sortie et d'entrée du ventilateur médical.
- la branche inspiratoire du circuit patient peut comprendre un humidificateur de gaz.
- l'humidificateur de gaz est agencé en aval du module d'injection de NO de manière à pouvoir humidifier le gaz avant son administration par inhalation au patient.

Selon un autre aspect, la présente divulgation concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO et au moins 20 %vol. d'oxygène, de préférence au moins 21 %vol. d'oxygène, au moyen d'une installation de fourniture de gaz, telle celle décrite ci-avant, comprenant un dispositif de délivrance de NO équipé du système de dosage de secours de NO selon l'invention, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les termes « concentration », « dose » et « teneur » sont considérés comme équivalents.
- les termes « pilotage », « commande » et « contrôle » sont considérés comme équivalents et substituables.
- les termes « moyen de/à/pour » sont considérés comme totalement équivalents et substituables par les termes « dispositif de/à/pour » ou des termes équivalents comme « unité », par exemple les termes « moyens de pilotage » peuvent être remplacés par « dispositif de pilotage » ou « unité de pilotage », les termes « moyens de mesure » peuvent être remplacés par « dispositif de mesure »...
- Par « fonctionnement normal » : on entend un fonctionnement habituel de l'appareil de délivrance de NO pendant une première période de temps (de durée non-nulle), en l'absence de toute panne, dysfonctionnement, défaut ou autre. La première période de temps a une durée de typiquement une à plusieurs minutes, voire heures ou jours, ou même plus.
- Par « dysfonctionnement », on entend une panne, une anomalie, un problème, un mauvais fonctionnement, un défaut ou analogue, qu'il soit électrique, mécanique ou d'une autre nature, affectant le fonctionnement normal de l'appareil de délivrance de NO, en particulier empêchant le fonctionnement des moyens de pilotage du dispositif, pendant une seconde période de temps (de durée non-nulle), par exemple du fait d'une panne des moyens de pilotage et/ou un défaut d'alimentation électrique de ceux-ci. La seconde période de temps a une durée variable, par exemple de quelques secondes à une ou plusieurs minutes, ou dizaine(s) de minutes, ou même plus.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de délivrance de gaz comprenant un dispositif de délivrance de NO équipé d'un système de dosage d'urgence de NO selon la présente invention.
Fig. 2 à Fig. 5 schématisent le fonctionnement de l'association orifice calibré/actionneur du système de dosage d'urgence de NO de Fig. 1.

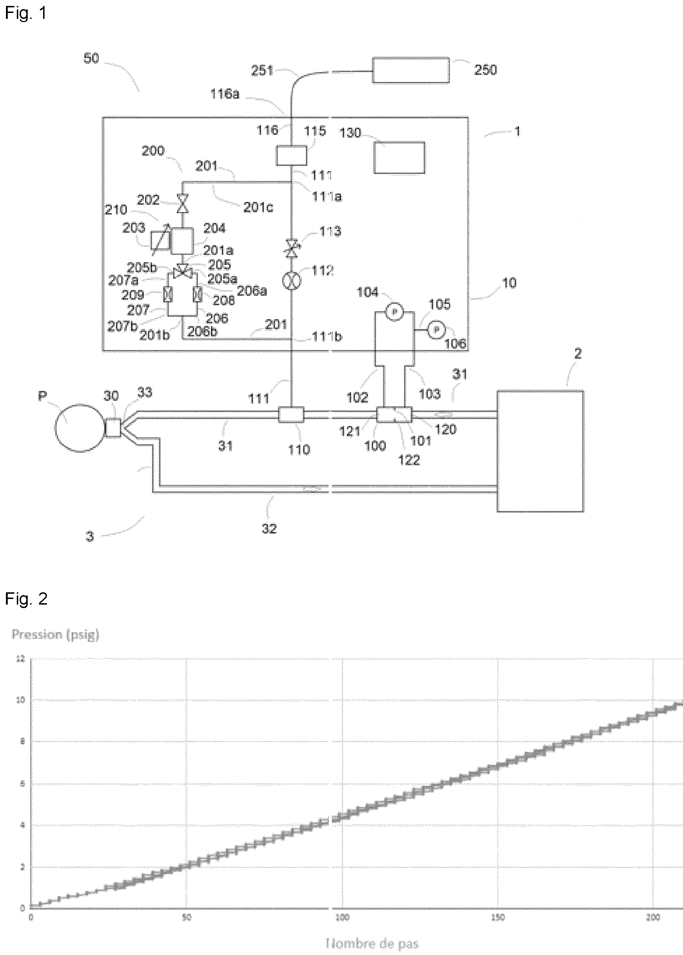

Fig. 1 schématise un mode de réalisation d'une installation de délivrance de gaz 50 selon la présente invention comprenant un appareil ou dispositif de délivrance de NO 1 comprenant un système de dosage d'urgence de NO, associé à un ventilateur mécanique 2, c'est-à-dire un appareil respiratoire délivrant un gaz respiratoire.

Cette installation 50 est configurée pour délivrer du NO sous forme gazeuse à un patient à une concentration désirée correspondant à une posologie fixée par un médecin-anesthésiste ou analogue, typiquement entre 1 et 80 ppmv de NO (i.e. ppm en volume), en particulier un flux de mélange NO/N₂.

Le ventilateur médical 2 délivre un gaz respiratoire contenant au moins 20 vol.% d'oxygène environ, de préférence au moins 21 vol.% d'oxygène environ, tel que de l'air ou un mélange O₂/N₂, dans un circuit patient 3, en particulier dans une branche inspiratoire 31 du circuit patient 3, servant à acheminer et à fournir le gaz à un patient P et à convoyer les gaz expirés par le patient via une branche expiratoire 32 du circuit patient 3.

Le ventilateur médical 2 est un appareil d'assistance respiratoire classique pouvant comprendre, selon le mode de réalisation désiré, soit une soufflante motorisée, aussi appelée turbine ou compresseur, soit une ou des vannes proportionnelles, en lieu et place de la soufflante motorisée, qui sont alimentées en gaz, par exemple en air médical, par une prise murale alimentée par un réseau hospitalier véhiculant le gaz au sein d'un établissement hospitalier.

Dans tous les cas, lorsque le ventilateur médical 2 délivre le gaz respiratoire dans le circuit patient 3, son fonctionnement est contrôlé par une (ou des) carte électronique de commande ou analogue agencée(s) dans le ventilateur médical 2. Il est alimenté électriquement par des moyens d'alimentation électrique, tel le secteur (110/220V) et/ou une batterie interne.

A titre d'exemple, le ventilateur médical 2 peut être le Servo-n Néonatal^{®} de la société Getinge, qui est un ventilateur à vannes proportionnelles incluant une fonction HFO. Bien entendu, un autre ventilateur médical 2 peut aussi convenir.

Comme visible sur Fig. 1, la branche inspiratoire 31 et la branche expiratoire 32 sont reliées fluidiquement à une pièce de jonction 33, telle une pièce en Y ou similaire, en communication fluidique avec une interface respiratoire 30 permettant de délivrer le gaz au patient P ou, à l'inverse, de collecter les gaz expirés par le patient P. L'interface respiratoire 30 peut par exemple être un masque facial, une sonde d'intubation trachéale ou autre.

Les branches inspiratoire 31 et expiratoire 32 comprennent des conduits, canalisations, tuyaux, passages, tubulures ou similaires, par exemple des tuyaux flexibles en polymère, aptes à et configurés pour convoyer les flux gazeux.

Le gaz respiratoire circule dans la branche inspiratoire 31 dans le sens allant du ventilateur mécanique 2 vers le patient P, alors que les gaz expirés enrichis en CO₂, circulent dans la branche expiratoire 32 en direction du ventilateur mécanique 2 où ils sont déchargés à l'atmosphère.

Un capteur de débit 100 et un module d'injection de NO 110 sont agencés dans la branche inspiratoire 31. Le capteur de débit 100 est généralement agencé entre le module d'injection de NO 110 et le ventilateur mécanique 2 de manière à pouvoir mesurer le débit de gaz provenant du ventilateur mécanique 2. La branche inspiratoire 31 peut également comprendre un humidificateur (non représenté) afin d'humidifier le gaz délivré au patient, lequel est préférentiellement agencé en aval du module d'injection de NO 110, c'est-à-dire entre le module d'injection de NO 110 et l'interface respiratoire 30, telle une sonde d'intubation trachéale.

Le capteur de débit 100 sert à mesurer le débit gazeux, e.g. air ou mélange O₂/N₂, fourni par le ventilateur mécanique 2 et circulant dans la branche inspiratoire 31. Les mesures opérées sont fournies, directement ou indirectement, aux moyens de pilotage 130 de l'appareil de délivrance de NO 1 qui les utilisent pour contrôler ou ajuster la quantité de NO apportée par le dispositif de délivrance de NO 1, c'est-à-dire le flux de NO, typiquement de mélange gazeux NO/N₂, fourni par l'appareil de délivrance de NO au module d'injection de NO 110, comme expliqué ci-après.

On peut utiliser par exemple un capteur de débit massique, un capteur à mesure de pression différentielle ou tout autre capteur adapté.

Dans le mode de réalisation de Fig. 1, le capteur de débit 100 est par exemple du type à mesure de pression différentielle, c'est-à-dire que le capteur de débit 100 comprend une restriction interne 101 qui crée une perte de charge engendrant un différentiel ou gradient de pression lorsqu'un débit gazeux traverse cette restriction interne 101. Le capteur de débit 100 comprend des chambres amont 120 et aval 121 qui sont séparées par une paroi 122 traversées un passage de gaz de sorte de former la restriction interne 101.

Des lignes de mesure de pression amont 103 et aval 102 sont connectées fluidiquement au capteur de débit 100 en des sites de raccordement situés en amont et en aval de la restriction interne 101, en particulier aux chambres amont 120 et aval 121, afin d'y opérer les mesures de pression du flux gazeux circulant, avant et après perte de charge, i.e. de l'air ou un mélange O₂/N₂.

La différence de pression créée par la restriction interne 101 est déterminée par un capteur de pression différentiel 104 raccordé au capteur de débit 100 par le biais des lignes de pression amont 102 et aval 103 qui forment des conduits de mesure de pression et fournissent au capteur de pression différentiel 104, les mesures de pression du flux circulant, avant et après perte de charge.

Préférentiellement, le capteur de pression différentiel 104 est intégré dans le boitier 10 du dispositif de délivrance de NO 1, comme illustré en Fig. 1.

Le capteur 104 est par ailleurs connecté électriquement à une unité de pilotage 130, aussi appelée contrôleur ou moyens de pilotage, et/ou lui transmet les mesures de pression afin qu'elles y soient traitées informatiquement, en particulier pour réguler oui ajuster le début de NO, typiquement de mélange NO/N₂, fourni par l'appareil de délivrance de NO 1 au module d'injection de NO 110.

Le module d'injection de NO 110 injecte le débit de NO, i.e. NO/N₂, dans le flux de gaz circulant dans la branche inspiratoire 31 pour y réaliser le mélange désiré, c'est-à-dire typiquement un mélange NO/O₂/N₂ contenant le NO à la concentration désirée correspondant à la posologie fixée par un médecin ou analogue, qui est typiquement entre 1 et 80 ppmv, en général entre 5 et 40 ppmv de NO, le reste étant de l'oxygène (>20 vol.% environ) et de l'azote, voire des impuretés inévitables (par exemple de l'argon...) et de la vapeur d'eau, en particulier lorsqu'un humidificateur est présent en aval du module d'injection de NO 110.

Avantageusement, on peut prévoir une ligne de dérivation 105 qui vient se connecter à la ligne de pression aval 103 afin de convoyer l'information de pression régnant dans ladite ligne de pression aval 103 à un capteur de pression 106, typiquement de type relatif. Ce capteur de pression 106 mesure la pression régnant dans la chambre amont 120 du capteur de débit 100 et peut retourner cette valeur, via une connexion électrique, à l'unité de pilotage 130 à des fins de compensation, considérant que la valeur de débit réelle traversant le capteur de débit 100 dépend principalement de la mesure de pression différentielle 104 mais est également affectée par la pression relative 106 régnant en amont du capteur de débit 100.

L'unité de pilotage 130 comprend un système de traitement de données, en particulier des mesures provenant des capteurs 100, 104, 106, comprenant typiquement un (ou des) microprocesseur(s) agencé sur une (ou des) carte électronique et mettant en œuvre un (ou des) algorithme(s), i.e. un (ou des) programme d'ordinateur. Bien entendu, l'unité de pilotage 130 peut être aussi configurée pour piloter d'autres éléments électromécaniques intégrés dans le boitier 10 du dispositif de délivrance de NO 1.

Plus précisément, l'unité de pilotage 130 est configurée pour traiter et/ou exploiter les mesures, c'est-à-dire les signaux de mesure de pression ou les valeurs de pression, transmises par le capteur de pression différentielle 104 coopérant avec le capteur de débit 100, et/ou par le capteur de pression 106. Avantageusement, l'unité de pilotage 130 dispose d'une table de correspondance préenregistrée, i.e. mémorisée, qui permet une détermination du débit de gaz circulant dans la branche inspiratoire 31, i.e. traversant le capteur de débit 100, c'est-à-dire de transformer une valeur de pression transmise par le capteur de pression 104, tel ici un capteur de pression différentiel, en valeur de débit traversant le capteur de débit 100, éventuellement compensée de la valeur retournée par le capteur de pression 106.

D'une façon générale, la détermination du débit du flux gazeux (e.g. air) traversant le capteur de débit 100 permet ensuite de calculer la quantité de NO (i.e. le débit de NO/N₂) devant être injectée au flux de gaz circulant dans la branche inspiratoire 31 par le module d'injection de NO 110 afin de pouvoir délivrer le NO au patient à la concentration désirée correspondant à la posologie fixée par un médecin-anesthésiste ou analogue, typiquement entre 1 et 80 ppmv de NO (i.e. ppm en volume).

En d'autres termes, en utilisant la mesure de pression retournée par le capteur de pression différentielle 104 et la table de correspondance mémorisée, l'unité de pilotage 130 peut déterminer le débit de gaz (e.g. air ou N₂/O₂ avec teneur en O₂ > 21 % vol.) issu du ventilateur mécanique 2 et la quantité de NO devant être ajoutée, via le module d'injection de NO 110, afin d'obtenir la concentration de NO souhaitée.

Comme déjà dit, le mélange gazeux final obtenu au niveau du module d'injection de NO 110 comprend alors principalement de l'azote (N₂), de l'oxygène (O₂) en une teneur d'au moins 20 à 21%vol., et du NO à une teneur typiquement comprise entre 1 et 80 ppmv, voire des impuretés inévitables et/ou de la vapeur d'eau, en particulier lorsqu'on humidificateur de gaz est présent.

Plus précisément, en fonction du débit gazeux (i.e. air ou N₂/O₂) circulant dans la branche inspiratoire 31 ayant été déterminé à l'aide du capteur de débit 100, l'unité de pilotage 130 détermine la quantité de NO, typiquement de mélange NO/N₂, devant être ajouté au gaz ayant une teneur en O₂ > 20% vol. (e.g. air ou N₂/O₂) circulant dans la branche inspiratoire 31 afin d'obtenir la concentration en NO finale souhaitée.

L'appareil de délivrance de NO 1 est alimenté en NO gazeux, typiquement en mélange NO/N₂ gazeux, provenant d'une source de NO 250 reliée fluidiquement au dispositif de délivrance de NO 1, en particulier à une ligne haute pression 116 dudit dispositif de délivrance de NO 1, par une ligne d'alimentation 251, tel un conduit flexible ou analogue. Typiquement, la source de NO 250 est une (ou des) bouteille(s) de gaz sous pression renfermant un mélange de NO/N₂ contenant une concentration en NO généralement comprise entre 100 et 30000 ppmv.

Le mélange NO/N₂ est fourni au module d'injection 110 par l'appareil de délivrance de NO 1, via une ligne d'injection 111, telle une conduite de gaz flexible, qui est fluidiquement connectée à la ligne haute pression 116 de l'appareil de délivrance de NO 1, laquelle comprend une entrée haute pression 116a connectée fluidiquement à la source de NO pour être alimentée en NO/N₂ sous pression, e.g. 10 bar abs.

La ligne haute pression 116, par exemple un passage ou conduit de gaz, comprend un régulateur de pression 115 qui réduit la pression du mélange NO/N₂ à une valeur stable, par exemple environ 2 bar abs ou toute autre pression adéquate. Le port de sortie du régulateur de pression 115 fourni donc une pression stable dans la portion amont de la ligne d'injection 111.

Un dispositif à vanne 113, telle une électrovanne, avantageusement une électrovanne proportionnelle, par exemple l'électrovanne de la série VSO miniature disponible auprès de la société Parker, est agencé dans l'appareil de délivrance de NO 1 afin de contrôler le débit de NO gazeux au sein de la ligne d'injection 111.

Le débit gazeux circulant dans la ligne d'injection 111 est mesuré par un dispositif de mesure de débit ou capteur de débit de NO 112, agencé sur la ligne d'injection 111, préférentiellement placé en aval du dispositif à vanne 113, comme visible sur Fig. 1.

Le régulateur de pression 115, le dispositif à vanne ou électrovanne 113, le capteur de débit de NO 112 et une portion amont de la ligne d'injection 111 sont donc agencés dans le boitier 10 de l'appareil de délivrance de NO 1.

Le dispositif à vanne 113 est configuré pour être normalement dans une position fermée (i.e. un état fermé) pour empêcher toute circulation de gaz dans la ligne d'injection 111. Pour passer en position ouverte, le dispositif à vanne 113 doit être commandé par les moyens de pilotage 130, comme c'est le cas pendant un fonctionnement normal de l'appareil de délivrance de NO 1.

Par ailleurs, on prévoit un système de dosage de secours de NO, c'est-à-dire un circuit de secours 200, agencé dans le boitier 10 de l'appareil de délivrance de NO 1, lequel est configuré pour fonctionner en cas de dysfonctionnement de l'appareil de délivrance de NO 1, comme expliqué ci-après.

Le circuit de secours 200 comprend (au moins) à une ligne de secours 201, aussi appelée ligne de bipasse, tel un passage ou un conduit de gaz, ou analogue.

Dans le mode de réalisation proposé en Fig. 1, la ligne de secours 201 du circuit de secours 200 vient se connecter fluidiquement à la ligne d'injection 111 en un premier site de raccordement 111a, i.e. en amont, situé entre le régulateur de pression 115 et le dispositif à vanne 113, et en un second site de raccordement 111b, i.e. en aval, situé en aval du dispositif à vanne 113 et, de préférence, en aval du capteur de débit de NO 112.

Autrement dit, le dispositif à vanne 113 et préférentiellement le capteur de débit de NO 112 sont situés entre les premier et second sites de raccordement 111a, 111b de la ligne de secours 201, c'est-à-dire que la ligne de secours 201 bipasse, le dispositif à vanne 113 et préférentiellement le capteur de débit de NO 112 agencés sur la ligne d'injection 111.

Alternativement, selon un autre mode de réalisation, le second site de raccordement 111b peut être situé entre le dispositif à vanne 113 et le capteur de débit de NO 112.

Dans tous les cas, le gaz circule dans la ligne de secours 201 dans le sens allant du premier site de raccordement 111a au second site de raccordement 111b.

Le circuit de secours 200 comprend aussi une électrovanne de secours 202 et un dispositif de contrôle de débit 210 qui sont agencés sur la ligne de secours 201 et qui servent à contrôler le débit de gaz au sein du circuit de secours 200, typiquement au sein de la ligne de secours 201.

Une électrovanne à 3 voies 205, c'est-à-dire de type 3:2, est agencée sur la ligne de secours 201, en aval du dispositif de contrôle de débit 210. Elle est contrôlée par les moyens de pilotage 130.

L'électrovanne à 3 voies 205 comprend un port amont 201a, un premier port aval 205a et un second port aval 205b, en communication fluidique. La sélection de la communication fluidique entre le port amont 201a et le premier port aval 205a ou, alternativement, le second port aval 205b est opérée par les moyens de pilotage 130, comme décrit ci-après.

L'électrovanne à 3 voies 205 est de type bistable, c'est-à-dire qu'en cas d'absence de commande électrique par les moyens de pilotage 130, par exemple en cas de dysfonctionnement, typiquement en cas de perte d'alimentation électrique, la communication fluidique existant entre le port amont 201a et le premier port aval 205a ou le second port aval 205b est conservée, c'est-à-dire qu'elle reste dans l'état dans lequel elle se trouvait avant dysfonctionnement.

A titre d'exemple, on peut utiliser l'électrovanne 205 référencée HDI disponible auprès de The Lee Company^{®}.

Le premier port aval 205a de l'électrovanne 205 est en communication fluidique avec une première ligne de dosage 206, dans laquelle est agencé un premier dispositif à orifice calibré 208, alors que le second port aval 205b de l'électrovanne 205 est en communication fluidique avec une seconde ligne de dosage 207 dans laquelle est agencé un second dispositif à orifice calibré 209.

Les premier et second dispositifs à orifice calibré 208, 209 présentent des caractéristiques différentes en termes de section de passage de leurs orifices calibrés respectifs, e.g. des diamètres différents. Ainsi, le premier orifice calibré du premier dispositif à orifice calibré 208 peut avoir un premier diamètre de passage D1 et le second dispositif à orifice calibré 209 présente un second diamètre de passage D2, tels que D1 < D2, de préférence 1,5.D1 < D2 < 4.D1.

Par exemple, le premier orifice calibré du premier dispositif à orifice calibré 208 peut avoir un premier diamètre de passage D1 de l'ordre de 25 µm et le second orifice calibré du second dispositif à orifice calibré 209 présente un second diamètre de passage D2 de l'ordre de 50 µm, c'est-à-dire que D2 est de préférence égal à environ 2.D1.

A titre d'exemple, on peut utiliser des calibrés disponibles auprès de la société O'Keefe Control^{®} sous les références BLP-1-SS et BLP-2-SS. Bien entendu, on peut utiliser d'autres orifices calibrés de diamètres et/ou de formes différentes.

En aval des premier et second dispositifs à orifices calibrés 208, 209, les première et seconde lignes de dosage 206, 207 se rejoignent en site de raccordement 201b, appelé nœud, et se raccordent par ailleurs, en ce même site 201b, à la partie aval de la ligne de secours 201, laquelle ligne de secours 201 vient se raccorder fluidiquement (en aval du site 201b) à la ligne d'injection 111 au niveau du second site de raccordement 111b. Autrement dit, la première ligne de dosage 206 et la seconde ligne de dosage 207 viennent se raccorder au niveau du nœud 201b de manière à former la partie aval de la ligne de secours 201.

L'électrovanne de secours 202 est configurée pour être normalement dans une position ouverte (i.e. état ouvert) pour permettre une circulation de gaz dans la ligne de secours 201, c'est-à-dire qu'elle laisse passer le flux gazeux lorsqu'elle n'est pas ou plus commandée par l'unité de pilotage 130. Pendant un fonctionnement normal du dispositif de délivrance de NO 1, l'électrovanne de secours 202 est donc commandée par les moyens de pilotage 130 pour être en position fermée (i.e. état fermé) pour empêcher que le flux de NO/N2 n'emprunte la ligne de secours 201.

L'électrovanne de secours 202 est préférentiellement une électrovanne de type « tout ou rien » ayant deux états possibles, à savoir un état ouvert laissant passer le flux gazeux et un état fermé ne laissant pas passer le flux gazeux. Elle est pilotée par l'unité de pilotage 130. On peut utiliser par exemple une électrovanne de la série Picosol de IMI Norgren^{®}, ou de la série HDI de The Lee Company ^{®}.

Comme déjà dit, l'électrovanne de secours 202 est normalement ouverte, c'est-à-dire qu'en l'absence d'une commande électrique venant de l'unité de pilotage 130, l'électrovanne de secours 202 est en état ouvert, i.e. position ouverte, ce qui permet alors au gaz issu de la source de NO d'emprunter la ligne de secours 201 depuis le premier site de raccordement 111a en direction du second site de raccordement 111b.

Par contre, l'unité de pilotage 130 commande la fermeture de l'électrovanne de secours 202, c'est-à-dire son passage de l'état ouvert à l'état fermé, i.e. en position fermée, on empêche toute circulation de gaz dans la ligne de secours 201, en particulier lorsqu'un flux n'y est pas souhaité, typiquement en fonctionnement normal.

Autrement dit, les moyens de pilotage 130, i.e. l'unité de pilotage, sont configurés pour coopérer avec l'électrovanne de secours 202, le dispositif de contrôle de débit 210, le dispositif à vanne 113 et le dispositif de mesure de débit 112, lors d'un fonctionnement normal du dispositif de délivrance de NO 1, afin de diriger le flux gazeux vers la ligne d'injection 111 et empêcher qu'il ne puisse circuler dans la ligne de secours 201, et inversement en cas de dysfonctionnement, comme expliqué ci-après.

Dans le mode de réalisation de Fig. 1, le dispositif de contrôle de débit 210 comprenant un moyen actionneur 203, de préférence à réglage par déplacement angulaire, typiquement un moteur pas à pas, coopérant avec un régulateur de pression pneumatique 204 et forme ainsi un système à pression variable permettant de contrôler le débit et la pression du flux gazeux.

Lorsque l'électrovanne de secours 202 est ouverte, c'est-à-dire non commandée par l'unité de pilotage 130, typiquement en cas de défaut d'alimentation ou de dysfonctionnement l'unité de pilotage 130, la pression régnant dans la portion amont 201c de la ligne de secours 201 (i.e. entre le premier site de raccordement 111a et le régulateur pneumatique 204) est égale à la pression de détente du régulateur de pression 115, par exemple ici égale à 2 bar rel. (14 psig). Cette même pression s'exerce aussi en entrée du régulateur pneumatique 204 qui est agencé sur la ligne de secours 201.

Le régulateur pneumatique 204 peut être, quant à lui, réglé sur plusieurs niveaux de pression différents, typiquement jusqu'à 2 bar relatif, par exemple entre 0 et 1.4 bar relatif environ (i.e. 0-20 psi), en fonction de la tension de son ressort interne. On peut par exemple utiliser un régulateur pneumatique disponible auprès de Beswick Engineering^{®} sous la référence PRDB.

Le moyen actionneur 203, à savoir ici un moteur pas à pas, est mécaniquement couplé au régulateur pneumatique 204 de manière à ce qu'une position angulaire donnée du moteur pas à pas, influe sur la tension du ressort interne du régulateur pneumatique 204 et fixe ainsi une pression de sortie en aval dudit régulateur pneumatique 204.

Autrement dit, le moteur pas à pas peut, en fonction de sa position angulaire, déterminée par les moyens de pilotage 130, piloter le régulateur pneumatique 204, en particulier en agissant sur la tension de son ressort interne, pour passer d'une position fermée, i.e. délivrant une pression nulle à sa sortie, à une position ouverte, délivrant une pression maximale à sa sortie, typiquement inférieure à 2 bar relatif, par exemple de l'ordre de 1.4 bar relatif (i.e. 20 psig environ).

Bien entendu, selon la position angulaire du moteur pas à pas, c'est-à-dire en fonction de son « nombre de pas », le régulateur pneumatique 204 peut adopter une (ou plusieurs, position intermédiaire, délivrant ainsi une pression comprise entre un minimum, par exemple 0 bar relatif, et un maximum typiquement inférieur à 2 bar relatif, par exemple 1.4 bar relatif.

Dès lors, la résolution en termes de pressions réglables en aval du régulateur pneumatique 204 dépend de la finesse et du nombre de pas définissant une position donnée du moteur pas à pas, i.e. du moyen actionneur 203, de sa position suivante.

Ainsi, Fig. 2 représente une évolution de la pression de sortie du régulateur pneumatique 204 en fonction d'un nombre de pas (i.e. position du moteur pas à pas), déterminé par les moyens de pilotage 130, qui permet de constater que la pression augmente de façon linéaire en fonction du nombre de pas. Plus précisément, on voit que la pression maximale de sortie du régulateur pneumatique 204 est limitée à 12 psig (830 mb rel.) mais qu'elle pourrait être plus élevée, typiquement jusqu'à 20 psig (1.4 bar rel.), en augmentant le nombre de pas.

Plus généralement, la pression en sortie du régulateur pneumatique 204 se retrouve alors au niveau du port amont 201a de l'électrovanne 205 qui est agencée sur la ligne de secours 201 en aval du régulateur pneumatique 204.

En fonctionnement normal, l'électrovanne à plusieurs voies 205, ici à trois voies, est par ailleurs commandée par les moyens de pilotage 130 pour réaliser une communication fluidique entre sa voie d'entrée qui est raccordée fluidiquement à la ligne de secours 201 en aval du dispositif de contrôle de débit 210, et l'une ou l'autre de sa première voie de sortie qui est raccordée fluidiquement à la première ligne de dosage 206, et de sa seconde voie de sortie qui est raccordée fluidiquement à la seconde ligne de dosage 207, c'est-à-dire entre son port amont 201a et l'un de ses ports aval 205a, 205b.

La pression de sortie du régulateur pneumatique 204 se propage alors dans la portion amont 206a, de la première ligne de dosage 206 située en amont du premier orifice calibré 208 ou, selon le cas, dans la portion amont 207a de la seconde ligne de dosage 207 située en amont du second orifice calibré 209.

Autrement dit, pendant le fonctionnement normal de l'appareil 1, les moyens de pilotage 130 contrôlent l'électrovanne à 3 voies 205 pour opérer une communication fluidique entre la voie d'entrée de ladite électrovanne 205 et l'une ou l'autre des première et seconde voies de sortie de l'électrovanne à 3 voies 205 de manière à faire circuler le flux gazeux dans l'une ou l'autre des lignes de dosage 206, 207, donc au travers du premier ou du deuxième dispositif à orifice calibré 208, 209 qui comprennent des sections de passage ou diamètres de leurs orifices calibrés D1, D2 différents.

Or, d'une façon générale, pour tout orifice calibré, il existe une relation entre la pression en amont de l'orifice calibré et le débit le traversant puisque le débit dépend des dimensions de l'orifice considéré. En effet, le débit traversant un orifice calibré est en relation avec le différentiel de pression existant entre la pression en amont et la pression en aval de cet orifice calibré.

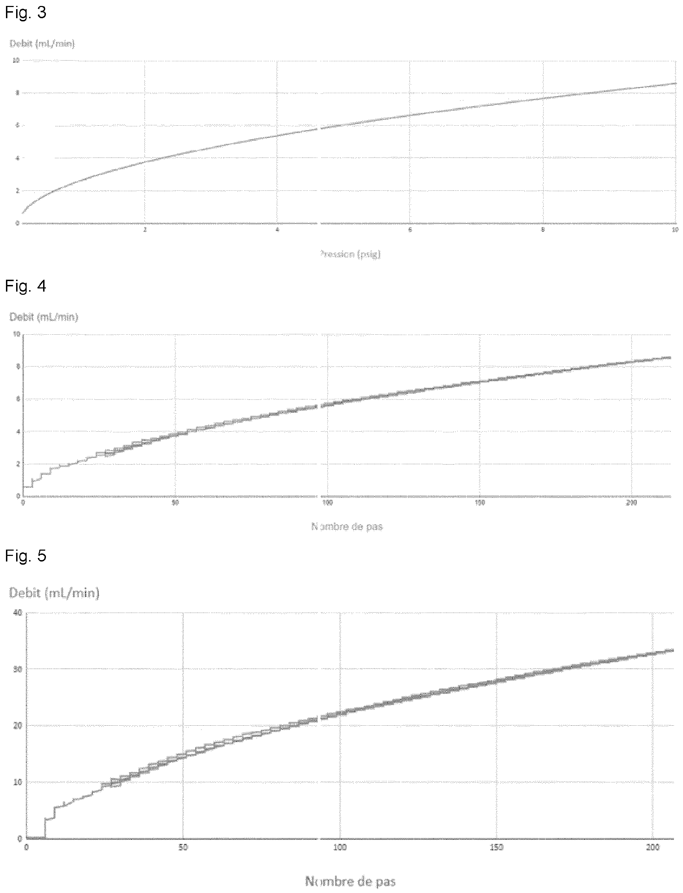

Ainsi, Fig. 3 représente la relation liant la pression (en psig) en amont du premier orifice du premier dispositif à orifice calibré 208 et le débit (en mL/min) le traversant, c'est-à-dire circulant dans la portion aval 206b de la première ligne de dosage 206.

On y voit que le débit augmente au fur et à mesure que la pression en amont augmente. Cette augmentation ne suit pas une loi linéaire mais plutôt de type « racine carrée », comme largement documenté dans la littérature.

Dès lors, ici, en fonction de la position de l'électrovanne 205, le débit traversant le premier orifice calibré du premier dispositif à orifice calibré 208 dépend de la différence des pressions régnant respectivement dans les portions amont 206a et aval 206b de la première ligne de dosage 206, et inversement, le débit traversant le second orifice calibré du second dispositif à orifice calibré 209 dépend de la différence des pressions régnant respectivement dans les portions amont 207a et aval 207b de la seconde ligne de dosage 207.

De préférence, la pression régnant en aval des premier et second orifices calibrés, c'est-à-dire en aval des deux dispositifs à orifice calibré 208, 209 (i.e. dans les portions aval 206b, 207b), est par ailleurs considérée comme étant négligeable. Toutefois, des moyens de mesure supplémentaires, tel qu'un dispositif de mesure de pression additionnel, peuvent être agencés pour opérer une mesure de pression en aval des premier et second orifice calibré, par exemple dans la région du au nœud 201b de la ligne de secours 201, et utilisés à des fins de compensation de pression afin d'accroitre la précision du dispositif de contrôle de débit 210, comme détaillé ci-après.

Par ailleurs, l'expression du débit correspondant aussi à une position du moteur pas à pas 203, exprimée sous forme de pas, comme représenté en Fig. 4, en s'appuyant sur la relation linéaire reliant la position du moteur 203 à la pression de sortie du régulateur pneumatique 204, comme illustré en Fig. 2.

Ainsi, Fig. 4 montre qu'un nombre de pas égal à 0 correspond à une position fermée du régulateur pneumatique 204 et chaque pas correspond à un changement de position du moteur pas à pas 203 ouvrant un peu plus le régulateur pneumatique 204 pour laisser passer le flux gazeux. Par exemple, pour 50 pas réalisés, on obtient un débit d'environ 4 ml/min, alors que pour 100 pas réalisés, le débit est d'environ 5.5 ml/min...

De là, en renseignant une table de correspondance liant un nombre de pas (i.e. une position du moteur pas à pas 203) et un débit résultant, les moyens de pilotage 130 peuvent « (pré-)régler » le système ou circuit de dosage de secours 200, pendant le fonctionnement normal de l'appareil 1, comme expliqué ci-après, afin qu'il soit opérationnel en cas de dysfonctionnement de l'unité de pilotage 130, typiquement en cas de défaut d'alimentation de l'unité de pilotage 130.

Autrement dit, pendant le fonctionnement normal de l'appareil 1, les moyens de pilotage 130 contrôlent le régulateur pneumatique 204 pour régler ou fixer la position du moteur pas à pas 203 sur un nombre pas déterminé correspondant un débit de gaz souhaité.

De façon analogue, pendant le fonctionnement normal de l'appareil 1, lorsque les moyens de pilotage 130 commandent l'électrovanne à 3 voies 205 pour réaliser une communication fluidique entre son port amont 201a et par exemple son second port aval 205b, il est possible d'établir, comme précédemment, une table de correspondance reliant un nombre de pas (i.e. une position du moteur pas à pas 203) et un débit gazeux résultant, circulant alors dans la portion aval 207b de la seconde ligne de dosage 207, comme illustré en Fig. 5.

Etant donné que le diamètre du second orifice calibré 209 est supérieur à celui du premier orifice calibré 208, le débit résultant à « pas » similaire (i.e. position similaire) est plus important. Par exemple, pour 50 pas réalisés, on obtient un débit d'environ 15 ml/min (4 ml/min pour le premier orifice calibré 208).

Autrement dit, en fonction de la configuration de l'électrovanne à 3 voies 205, l'unité de pilotage 130 peut disposer d'une table de correspondance reliant un niveau de commande (i.e. de pas) donné à un débit de gaz traversant le premier ou, alternativement, le second orifice calibré 208, 209 en direction de la ligne d'injection 111 et y pénétrant au second site de raccordement 111b.

L'ensemble de ces (pré-)réglages est effectué pendant le bon fonctionnement de l'appareil 1, c'est-à-dire pendant son fonctionnement normal avant tout dysfonctionnement de l'unité de pilotage 130, en particulier lorsqu'elle n'est plus alimentée en courant électrique, donc ne fonctionne plus.

Ceci est ensuite mis à profit pour assurer une délivrance d'un débit de NO de secours (i.e. flux de NO/N₂), même en cas de dysfonctionnement de l'unité de pilotage 130, c'est-à-dire lorsqu'elle n'est plus alimentée en courant électrique, donc ne fonctionne plus, puisque tous les réglages ont déjà été faits, avant le dysfonctionnement.

Ainsi, en fonctionnement normal de l'appareil de délivrance de NO 1, c'est-à-dire lorsque l'unité de pilotage 130 est opérationnelle et normalement alimentée en courant électrique, l'électrovanne de secours 202 est commandée par l'unité de pilotage 130 pour être fermée, ce qui empêche toute circulation de gaz dans le circuit de secours 200 de Fig. 1, alors qu'en cas de dysfonctionnement de l'appareil 1 rendant l'unité de pilotage 130 non-opérationnelle, tel un défaut électrique, l'électrovanne de secours 202 ne peut plus être commandée par l'unité de pilotage 130 et donc s'ouvre pour laisser passer le gaz dans le circuit de secours 200, alors que l'électrovanne 113 se ferme, comme déjà expliqué. Le flux de gaz circulant dans le circuit de secours 200 est alors soumis aux (pré-)réglages faits, avant le dysfonctionnement, i.e. position du moteur pas à pas, envoi du flux vers le premier ou le second dispositif à orifice calibré 208, 209...

D'une façon générale, utiliser un moteur pas à pas en tant que moyen actionneur 203 est particulièrement recommandé car, contrairement aux électrovannes 202, 113 qui prennent une position de repos, en cas de coupure d'alimentation, à savoir une position ouverte pour l'électrovanne tout ou rien 202 et une position fermée pour l'électrovanne proportionnelle 113, la position du moteur pas à pas ne change pas, i.e. reste permanente, et fixée selon la dernière commande imposée et ce, indépendamment de toute alimentation électrique.

Autrement dit, la tension du ressort interne du régulateur pneumatique 204 présente une valeur fixe et égale à la dernière valeur de commande provenant des moyens de pilotage 130 et reçue par le moteur pas à pas, c'est-à-dire une position donnée correspondant à un nombre de pas donné, pendant le fonctionnement normal de l'appareil 1.

En cas d'alimentation en gaz du régulateur pneumatique 204, c'est-à-dire lorsque l'électrovanne de secours 202 s'ouvre du fait d'une absence de commande par les moyens de pilotage 130, la tension du ressort interne du régulateur pneumatique 204 engendre une pression fixe en aval du régulateur pneumatique 204, qui se retrouve alors au niveau du port amont 201a de l'électrovanne 205.

Bien entendu, la présente invention ne se limite pas à un actionneur de type moteur pas à pas. En effet, tout autre actionneur gardant sa position en cas de défaut d'alimentation électrique et pouvant être couplé à un mécanisme mécanique permettant de définir ou constituer un système à pression variable peut être utilisé, comme par exemple un moteur linéaire ou autre.

D'une façon générale, pendant son fonctionnement normal, le dispositif de délivrance de NO 1 est par ailleurs alimenté électriquement par une alimentation électrique, tel le secteur (110/220V) ou une batterie interne, afin de permettre le bon fonctionnement de ses composants nécessitant du courant électrique pour fonctionner, notamment l'actionneur 203, tel un moteur électrique pas à pas, l'unité de pilotage 130, les électrovannes 202, 113, 205 ou autres.

En outre, le dispositif de délivrance de NO 1 comprend aussi des moyens de mémorisation, telle une mémoire informatique, pour mémoriser des données, informations ou autres, par exemple une ou des tables de correspondances, comme expliqué ci-avant, les mesures de débit de gaz opérées par le dispositif de mesure de débit 112, ou autres.

De manière générale, en cas de défaillance majeure du fonctionnement du dispositif de délivrance de NO 1, tel un défaut d'alimentation électrique, par exemple causé par une rupture de son câble d'alimentation électrique occasionnée par des vibrations lors d'un transport de patient par exemple, on doit pouvoir continuer à assurer un traitement du patient avec du NO inhalé et ce, malgré le dysfonctionnement engendrant un arrêt de fonctionnement des moyens de pilotage 130, du fait par typiquement d'un défaut d'alimentation électrique.

A cette fin, le dispositif 1 de l'invention est configuré pour, qu'en cas d'une telle défaillance, l'électrovanne de secours 202 passe en position ouverte pour permettre une circulation de gaz dans la ligne de secours 201, alors que, dans le même temps, le dispositif à vanne 113 passe en position fermée pour stopper toute circulation de gaz dans la ligne d'injection 111, ce qui permet de fournir, via la ligne de secours 201 et le dispositif de contrôle de débit 210, le gaz à un débit gazeux de secours préfixé.

En fait, pendant le fonctionnement normal du dispositif 1 précédant le dysfonctionnement, ledit débit gazeux de secours est déterminé par les moyens de pilotage 130 à partir d'une ou des mesures de débit du gaz fournies par le dispositif de mesure de débit 112, aux moyens de pilotage 130. Les moyens de pilotage 130 peuvent alors prérégler le dispositif de contrôle de débit 210 et l'électrovanne 205 de sorte qu'ils puissent délivrer le gaz au débit gazeux de secours préfixé.

Autrement dit, les moyens de pilotage 130 déterminent le débit gazeux de secours devant être administré en cas de panne ou autre dysfonctionnement, à partir des mesures de débit du gaz fournies par le dispositif de mesure de débit 112 lors du fonctionnement normal du dispositif 1, et agissent sur le dispositif de contrôle de débit 210 et l'électrovanne à 3 voies 205 pour y régler ce débit gazeux de secours préfixé, par exemple en jouant sur le régulateur pneumatique 204, comme expliqué ci-avant.

Plus généralement, le fonctionnement de l'installation de délivrance de gaz 50 comprenant le dispositif de délivrance de NO 1 de l'invention est globalement le suivant.

Comme illustré en Fig. 1, le dispositif de délivrance de NO 1 coopère avec un ventilateur mécanique 2 afin d'apporter une aide thérapeutique au patient P. Comme déjà expliqué, le débit de gaz (i.e. air ou N₂/O₂) issu du ventilateur mécanique 2 et circulant dans la branche inspiratoire 31 du circuit patient 3 est mesuré en permanence par le capteur de débit 100 et l'unité de pilotage 130. La (les) mesure de débit opérée par le capteur de débit 100 permet à l'unité de pilotage 130 de déterminer, en temps réel, le débit de NO devant circuler dans la ligne d'injection 111 jusqu'au module d'injection de NO 110 afin d'injecter la quantité de NO au flux d'air provenant du ventilateur 2 de sorte de pouvoir obtenir la concentration finale en NO désirée, typiquement entre 5 et 80 ppmv, dans le mélange gazeux final NO/O₂/N₂ administré au patient P.

En fonctionnement normal, c'est-à-dire hors panne ou dysfonctionnement, afin de ne pas introduire de débit additionnel provenant de la ligne de secours 201 dans la ligne d'injection 111, l'unité de pilotage 130 commande l'électrovanne 202, qui est préférentiellement de type tout ou rien, en position fermée et, en parallèle, va piloter l'actionneur 203, tel un moteur pas à pas, afin de prérégler le régulateur pneumatique 204 en définissant un niveau de tension de son ressort interne, de préférence en fonction de la position adoptée par le moteur pas à pas 203, comme expliqué ci-avant.

Ceci est opéré par l'unité de pilotage 130 à partir d'une ou plusieurs mesures de débit provenant du dispositif de mesure de débit 112.

Plus précisément, l'unité de pilotage 130 réalise d'abord une moyenne du débit de NO (i.e. du mélange NO/N₂) ayant circulé dans la ligne d'injection 111 pendant un temps donné, par exemple pendant 1 minute ou sur une période de temps plus longue (mais le débit doit alors être converti en L/min ou bien en ml/min), pendant le fonctionnement normal du dispositif 1.

L'unité de pilotage 130 estime donc une valeur de débit de NO moyen fixe (en L/min ou ml/min) permettant de se rapprocher de la concentration en NO souhaitée.

Ainsi, dans Tab.1, on donne le débit de NO moyen fixe (en ml/min) pour différentes concentrations en NO sélectionnées (en ppmv), i.e. posologies, résultant de différentes ventilation minutes (L/min) mesurées par le capteur de débit 100, dont se servent les moyens de pilotage 130 pour déterminer le débit de NO à délivrer en temps réel.

**Tab. 1**

| | Ventilation Minute (L/min) | | | |
|---|---|---|---|---|
| Teneur en NO (posologie) (en ppmv) | 2 | 4 | 6 | 15 |
| 5 | 0,1 | 0,2 | 0,3 | 0,8 |
| 10 | 0,5 | 1 | 1,5 | 3,8 |
| 20 | 1 | 2 | 3 | 7,5 |
| 40 | 2 | 4 | 6 | 15 |
| 60 | 4 | 8 | 12 | 30,1 |
| 80 | 6 | 12 | 18,1 | 45,1 |

On constate que pour une ventilation minute moyenne de 2 L/min mesurée par le capteur de débit 100, et pour une posologie en NO de 5 ppmv, le débit de NO moyen est de 0.1 ml/min, et augmente lorsque la ventilation minute augmente et/ou la posologie en NO augmente. Le débit de NO moyen peut dès lors varier de 0.1 à 45 ml/min.

Afin de prendre en compte cette amplitude importante de débit, comme déjà mentionné, le système de dosage de secours 200 est pourvu d'un premier et d'un second dispositif à orifice calibré 208, 209, agencés sur les première et seconde lignes de dosage 206, 207 agencées en aval du régulateur pneumatique 204.

Comme illustré en Fig. 4, le premier dispositif à orifice calibré 208 est configuré pour générer des débits relativement faibles sur une large plage de pression, par exemple des débits inferieurs à 10 ml/min, alors que le second dispositif à orifice calibré 209 est configuré pour générer des débits plus importants pouvant excéder 50 ml/min, comme illustré en Fig. 5.

Or, sur la plage 0-10 ml/min, on remarque que la relation entre le débit et la position du moteur pas à pas est défavorable au dispositif à second orifice calibré 209 car une faible variation de position du moteur pas à pas 203 engendre une variation importante de débit, ce qui peut nuire à la précision des débits générés.

Dès lors, on configure le système pour que le flux gazeux passe par le premier dispositif à orifice calibré 208 lorsque le débit de NO moyen est faible, c'est-à-dire inférieur ou égal à 10 ml/min, et par le second dispositif à orifice calibré 209, pour des débits de NO moyen plus importants, c'est-à-dire supérieurs à 10 ml/min.

L'unité de pilotage 130 opère dès lors une commande spécifique de l'électrovanne à 3 voies 205 en fonction du débit de NO moyen pour opérer une communication fluidique entre son port amont 201a et son premier port aval 205a (si débit < 10 ml/min) ou, le cas échéant, son second port aval 205b (si débit > 10 ml/min), afin d'orienter le débit de NO de secours vers la première ligne de dosage 206 au travers du premier dispositif à orifice calibré 208 ou, selon le cas, vers la seconde ligne de dosage 207 au travers du second dispositif à orifice calibré 209.

En fonctionnement normal, l'unité de pilotage 130 réalise une moyenne du débit de NO et utilise ensuite la valeur ainsi déterminée pour commander l'électrovanne à 3 voies 205 pour sélectionner la première ou la seconde ligne de dosage 206, 207, donc le premier ou le second dispositif à orifice calibré 208, 209, destinée à prendre en charge le flux de NO, c'est-à-dire qui sera utilisée pour opérer le dosage de secours de NO, en cas de dysfonctionnement de l'appareil de NO 1, en particulier en cas de défaut d'alimentation électrique du capteur de débit 100 ou de l'unité de pilotage 130.

Par ailleurs, l'unité de pilotage 130 réalise une conversion par le biais d'une table de correspondance mémorisée ou analogue, en prenant en compte l'orifice calibre sélectionné, i.e premier ou second dispositif à orifice calibré 208, 209, de manière à commander l'actionneur 203 du dispositif de contrôle de débit 210, tel un moteur pas à pas, et définir un niveau de tension du ressort interne du régulateur pneumatique 204 afin d'autoriser un débit de NO circulant dans la ligne de secours 201 du système de secours 200 qui soit égal à la valeur calculée de NO moyen fixe. Cette valeur calculée de NO moyen sert donc de débit de NO gazeux de secours en cas de dysfonctionnement de l'appareil 1.

En fonctionnement normal, aucun débit de gaz ne circule dans la ligne de secours 201 car l'électrovanne tout ou rien 202 est fermée. Le flux de gaz circule normalement dans la ligne d'injection 111, via l'électrovanne proportionnelle 113 et le dispositif de mesure de débit 112, avant d'être fourni au module d'injection de NO 110, qui opère le mélange entre le flux de NO et le flux d'air ou analogue provenant du ventilateur 2.

Dès lors, en cas de défaillance majeure du dispositif de délivrance de NO 1 et/ou d'interruption de son alimentation électrique, à l'exception de l'actionneur 203, de l'électrovanne 205 et du régulateur de pression 115 qui a un fonctionnement purement pneumatique, l'ensemble des actionneurs électromécaniques, en particulier les électrovannes, retournent à leur position de repos, du fait que l'unité de pilotage 130 est, elle aussi, non alimentée. Par ailleurs, les différents capteurs se retrouvent sans alimentation électrique, donc sans capacité à communiquer et/ou à piloter/commander d'autres composants.

Ainsi, l'électrovanne proportionnelle 113 retrouve sa position de repos, à savoir sa position fermée empêchant tout passage de gaz, alors que l'électrovanne 202 se retrouve simultanément dans sa position de repos, à savoir sa position ouverte, autorisant ainsi le passage du gaz provenant de la source de NO dans la ligne de secours 201 du circuit de secours 200, et sa circulation jusqu'à atteindre le second site de jonction 111b, puis la partie aval de la ligne d'injection 111.

Le mélange NO/N₂ circule alors dans la ligne de secours 201 au débit de secours préfixé qui est contrôlé par l'association du régulateur pneumatique 204, en particulier par sa pression générée, et l'orifice calibré sélectionné, c'est-à-dire le premier dispositif à orifice calibré 208 ou le second dispositif à orifice calibré 209, sachant que, comme déjà expliqué, la dernière valeur valide du débit de NO moyen fixe a été alors déterminée par l'unité de pilotage 130 lors du fonctionnement normal du dispositif 1, préalablement à son dysfonctionnement.

Le débit de secours de NO/N₂ qui rejoint la ligne d'injection 111 (en 111b) peut ensuite être injecté dans la branche inspiratoire 31 du circuit patient 3 via le module d'injection de NO 110, comme déjà mentionné.

D'une façon générale, selon l'invention, le dispositif de contrôle de débit 210 est configuré pour fournir le gaz, i.e. NO/N₂, à un débit gazeux de secours préfixé, où ledit débit gazeux de secours est déterminé par les moyens de pilotage 130 à partir d'une (ou plusieurs) mesure de débit du gaz fournie par le dispositif de mesure de débit 112, pendant un fonctionnement normal du dispositif 1 précédant le dysfonctionnement, par exemple la dernière valeur de débit ayant été mesurée avant le dysfonctionnement affectant le bon fonctionnement du dispositif 1.

La valeur de débit est préréglée au sein du dispositif de contrôle de débit 210, par exemple en agissant sur la tension du ressort interne du régulateur pneumatique 204 du dispositif de contrôle de débit 210 comme expliqué ci-avant, par commande, c'est-à-dire préréglage, du dispositif de contrôle de débit 210 par les moyens de pilotage 130. Le préréglage a lieu, c'est-à-dire est opéré ou réalisé, pendant le fonctionnement normal du dispositif 1.

Bien entendu, en cas d'utilisation du circuit de secours 200 en cas de dysfonctionnement de l'appareil de délivrance de NO 1, on ne garantit pas une même précision de concentration en NO inhalé que lorsque le système de délivrance de NO 1 opère en fonctionnement normal, c'est-à-dire en ajustant le débit de NO en fonction du débit traversant le capteur de débit 100, mais cela évite une rupture de fourniture de NO au patient et, par ailleurs, le volume tampon généré par la portion de la branche inspiratoire 31 située en aval du module d'injection de NO, qui est éventuellement augmentée du volume de la chambre d'humidification lorsqu'elle est présente, permet de lisser les variations de concentration de NO inhalé par le patient et de se rapprocher de la valeur cible souhaitée, c'est-à-dire la posologie en NO.

Le système de dosage de secours de l'invention est donc particulièrement intéressant à mettre en œuvre car augmente la sécurité pour le patient qui ne risque pas d'être privé de son traitement par NO en cas de dysfonctionnement de l'appareil de délivrance de NO, et est reçoit une dose de NO très proche, voire égale, à la posologie recherchée.

Autrement dit, pouvoir s'approcher de la valeur cible de NO souhaitée grâce au système de dosage de secours 200 de NO intégré au dispositif de délivrance 1 de NO de l'invention améliore considérablement la sécurité pour le patient en comparaison avec un débit de NO de secours fixe usuellement délivré par le système de sécurité des dispositifs de délivrance de NO de l'art antérieur.

Ainsi, à titre comparatif, avec un système de secours basé sur un débit fixe, tel que classiquement mis en œuvre dans les dispositifs de délivrance de NO de l'art antérieur :
- pour un débit moyen de NO nécessaire de 0.05 L/min pour assurer normalement une concentration en NO de 10 ppmv (cas d'utilisation en néonatologie avec ventilateur de type HFO), la concentration résultante avec le débit fixe est de 50 ppmv, ce qui correspond à une multiplication par 5 de la posologie souhaitée.
- à l'inverse, pour un débit moyen nécessaire de NO de 1 L/min pour assurer 80 ppmv de concentration en NO (cas d'utilisation chez l'adulte, par exemple en cas d'hypertension pulmonaire pendant une chirurgie cardiaque), la concentration résultante chute à 20 ppmv, ce qui correspond à une diminution de 75% de la posologie souhaitée.

Dans les deux cas, les écarts importants de posologie peuvent induire des situations inacceptables et dangereuses pour le patient et ce, contrairement au système de dosage de secours 200 de NO intégré au dispositif de délivrance 1 de NO de l'invention qui permet lui de respecter la posologie désirée.

Il s'ensuit que le système de dosage de secours 200 de NO de l'invention présente des avantages indéniables en renforçant la sécurité des patients en :
- injectant de façon automatique un débit de secours de NO sans attendre que l'utilisateur ne se rende compte de la situation et intervienne en basculant sur le dosage pneumatique de secours.
- garantissant que la concentration de NO inhalé par le patient est similaire à la concentration souhaitée par le médecin, c'est-à-dire la posologie désirée.

Bien entendu, le basculement sur le système de dosage de secours 200 de NO de l'invention n'est que temporaire, c'est-à-dire ne dure que le temps nécessaire au remplacement de l'équipement ou composant défaillant qui a déclenché le système d'alarme sonore et/ou visuel afin d'alerter le personnel soignant.

Afin d'éviter l'activation à mauvais escient du système de dosage de secours 200 de NO, l'unité de pilotage 130 est en outre configurée pour procéder à des séquences d'initialisation et d'extinction adéquates. Par exemple, en cas d'arrêt voulu par l'utilisateur de la thérapie de NO, l'unité de pilotage 130 peut commander l'actionneur 203 afin de fermer le régulateur de pression 204. Ainsi, en cas d'extinction volontaire et donc d'ouverture de l'électrovanne 202, la configuration « fermée » du régulateur de pression 204 interdit alors toute circulation de débit de NO dans la ligne de secours 201, le temps que le dispositif de délivrance de NO 1 soit à l'arrêt.

Le dispositif de délivrance de NO 1 équipé du système de dosage de secours 200 de NO de l'invention est particulièrement bien adaptée à la fourniture de mélange gazeux comprenant de 1 à 80 ppmv de NO et au moins 20%vol. d'oxygène à des patients (adultes, enfants, adolescents ou nouveau-nés), de préférence au moins 21%vol. d'oxygène, souffrant d'hypertensions pulmonaires et/ou d'hypoxie, qui peuvent engendrer des vasoconstrictions pulmonaires ou analogues, par exemple causés par des pathologies ou troubles pulmonaires de type PPHN (hypertension pulmonaire persistante du nouveau-né) ou SDRA (syndrome de détresse respiratoire aigüe), ou encore engendrés par une opération de chirurgie cardiaque avec mise du patient sous circulation sanguine extracorporelle.

## Revendications

1. Appareil de délivrance de NO (1) pour fournir un gaz contenant du NO, en particulier un mélange gazeux NO/N₂, comprenant :
- une ligne d'injection de NO (111) pour acheminer le gaz contenant du NO,
- un dispositif à vanne (113) agencé sur la ligne d'injection (111) pour contrôler la circulation du gaz contenant du NO dans la ligne d'injection (111), ledit dispositif à vanne (113) étant configuré pour être normalement dans une position fermée pour empêcher toute circulation de gaz dans la ligne d'injection (111),
- un dispositif de mesure de débit (112) agencé sur la ligne d'injection (111) pour opérer une ou des mesures de débit du gaz contenant du NO circulant dans la ligne d'injection (111),
- un circuit de secours (200) comprenant une ligne de secours (201) venant se raccorder fluidiquement à la ligne d'injection (111), en amont (111a) et en aval (111b) du dispositif à vanne (113), ladite ligne de secours (201) comprenant une électrovanne de secours (202) configurée pour être normalement dans une position ouverte pour permettre une circulation de gaz dans la ligne de secours (201), et un dispositif de contrôle de débit (210), et
- des moyens de pilotage (130) configurés pour coopérer avec l'électrovanne de secours (202), le dispositif de contrôle de débit (210), le dispositif à vanne (113) et le dispositif de mesure de débit (112),
et dans lequel, en cas de dysfonctionnement engendrant un arrêt de coopération avec les moyens de pilotage (130) :
- l'électrovanne de secours (202) est configurée pour passer en position ouverte pour permettre une circulation de gaz dans la ligne de secours (201) du circuit de secours (200),
- le dispositif à vanne (113) est configuré pour passer en position fermée pour stopper toute circulation de gaz dans la ligne d'injection (111), et
- le dispositif de contrôle de débit (210) est configuré pour fournir le gaz à un débit gazeux de secours préfixé, où ledit débit gazeux de secours :
∘ est déterminé par les moyens de pilotage (130) à partir d'au moins une mesure de débit du gaz fournie par le dispositif de mesure de débit (112), pendant un fonctionnement normal de l'appareil (1) précédant ledit dysfonctionnement, et
∘ est préréglé par commande dudit dispositif de contrôle de débit (210) par les moyens de pilotage (130), pendant ledit fonctionnement normal de l'appareil (1),
**caractérisé en ce que** :
- une électrovanne à plusieurs voies (205) est agencée sur la ligne de secours (201), en aval du dispositif de contrôle de débit (210),
- ladite électrovanne à plusieurs voies (205) comprenant :
∘ une voie d'entrée raccordée fluidiquement à la ligne de secours (201) en aval du dispositif de contrôle de débit (210),
∘ une première voie de sortie raccordée fluidiquement à une première ligne de dosage (206) comprenant un premier dispositif à orifice calibré (208), et
∘ une seconde voie de sortie raccordée fluidiquement à une seconde ligne de dosage (207) comprenant un second dispositif à orifice calibré (209),
- la première ligne de dosage (206) et la seconde ligne de dosage (207) viennent se raccorder (201b) à la ligne de secours (201), en aval desdits premier et second dispositif à orifice calibré (208, 209),
- les premier et second dispositifs à orifice calibré (208, 209) présentent des orifices calibrés (D1, D2) de sections de passage ou diamètres différents,
- et les moyens de pilotage (130) sont configurés pour piloter l'électrovanne à plusieurs voies (205) pour diriger le flux gazeux vers la première ligne de dosage (206) ou, alternativement, vers la seconde ligne de dosage (207) en fonction de la ou des mesures de débit de gaz fournies par le dispositif de mesure de débit (112) lors du fonctionnement normal du dispositif (1) précédant ledit dysfonctionnement.

2. Appareil selon la revendication 1, **caractérisé en ce que** l'électrovanne à plusieurs voies (205) comprend 3 voies.

3. Appareil selon la revendication 1, **caractérisé en ce que** le premier orifice calibré du premier dispositif à orifice calibré (208) présente un premier diamètre de passage (D1) et le second orifice calibré du second dispositif à orifice calibré (209) présente un second diamètre de passage (D2) tels que 1,5.D1 < D2 < 4.D1.

4. Appareil selon la revendication 3, **caractérisé en ce que** le premier diamètre de passage (D1) et le second diamètre de passage (D2) sont tels que : 1,8.D1 < D2 < 3.D1, de préférence D2 est égal à environ 2.D1.

5. Appareil selon la revendication 1, **caractérisé en ce que** l'électrovanne de secours (202) est du type tout ou rien pouvant adopter uniquement un état ouvert dans lequel elle laisse passer le flux gazeux et un état fermé dans lequel elle interrompt le passage de flux gazeux.

6. Appareil selon la revendication 1, **caractérisé en ce que** le dispositif de contrôle de débit (210) comprenant un moyen actionneur (203) coopérant avec un régulateur de pression pneumatique (204), de préférence le moyen actionneur (203) comprend un moteur pas à pas.

7. Appareil selon la revendication 6, **caractérisé en ce que** :
- le régulateur pneumatique (204) est configuré pour être réglable sur plusieurs niveaux de pression compris entre 0 et 2 bar relatif, de préférence moins de 1,5 bar relatif, et
- le moyen actionneur (203) coopère avec le régulateur pneumatique (204) pour fixer une pression de sortie souhaitée en aval dudit régulateur pneumatique (204).

8. Appareil selon la revendication 7, **caractérisé en ce que** :
- le régulateur pneumatique (204) comprend un ressort interne permettant de régler le niveau de pression souhaité et
- le moyen actionneur (203) comprend un moteur pas à pas configuré pour adopter plusieurs positions angulaires différentes, chaque position angulaire du moteur pas-à-pas correspondant à une tension donnée du ressort interne du régulateur pneumatique (204),
de sorte que le niveau de pression de sortie souhaité en aval du régulateur pneumatique (204) soit déterminé par la tension du ressort interne du régulateur pneumatique (204) correspondant à la position angulaire adoptée par le moteur pas-à-pas.

9. Appareil selon la revendication 1, **caractérisé en ce qu'**en fonctionnement normal, les moyens de pilotage (130) sont configurés pour piloter l'électrovanne à plusieurs voies (205) pour opérer une communication fluidique entre la voie d'entrée de l'électrovanne à plusieurs voies (205) et l'une ou l'autre des première et seconde voies de sortie de l'électrovanne à plusieurs voies (205) de manière à faire passer le flux gazeux au travers du premier ou du deuxième dispositif à orifice calibré (208, 209).

10. Appareil selon la revendication 1, **caractérisé en ce que** les moyens de pilotage (130) comprennent au moins un microprocesseur.

11. Appareil selon la revendication 1, **caractérisé en ce que** le dispositif à vanne (113) comprend une électrovanne proportionnelle.

12. Appareil selon la revendication 1, **caractérisé en ce que** le dispositif de mesure de débit (112) est configuré pour opérer plusieurs mesures de débit successives, pendant le fonctionnement normal de l'appareil de délivrance de NO (1).

13. Appareil selon les revendications 1 et 6, 7 ou 8, **caractérisé en ce que** les moyens de pilotage (130) sont configurés pour déterminer l'ouverture du régulateur de pression pneumatique (204) et/ou le débit de secours à partir d'une table de correspondance mémorisée par des moyens de mémorisation, telle une mémoire informatique.

14. Installation de fourniture de gaz (1, 2) à un patient comprenant :
- au moins une source de NO (250) contenant un mélange gazeux NO/N₂, de préférence un mélange gazeux NO/N₂ contenant entre 100 et 2000 ppmv de NO, le reste étant de l'azote (N₂),
- un appareil de délivrance de NO (1) selon l'une des revendications précédentes, alimenté en mélange gazeux NO/N₂ par ladite au moins une source de NO (250),
- une branche inspiratoire (31) d'un circuit patient (3) alimentée en mélange gazeux NO/N₂ par l'appareil de délivrance de NO (1), et
- un ventilateur médical (2) en communication fluidique avec la branche inspiratoire (31) pour alimenter ladite branche inspiratoire (31) en un gaz respiratoire contenant au moins 20% d'oxygène, de préférence de l'air ou un mélange oxygène/azote.

## Patentansprüche

1. NO-Abgabevorrichtung (1) zur Abgabe eines NO-haltigen Gases, insbesondere eines NO/N₂-Gasgemisches , umfassend:
- eine NO-Einspritzleitung (111) zum Befördern des NO-haltigen Gases,
- eine Ventilvorrichtung (113), die an der Einspritzleitung (111) angeordnet ist, um den Durchfluss des NO-haltigen Gases in der Einspritzleitung (111) zu steuern, wobei die Ventilvorrichtung (113) so konfiguriert ist, dass sie sich normalerweise in einer geschlossenen Position befindet, um jeglichen Gasdurchfluss in der Einspritzleitung (111) zu verhindern,
- eine Durchflussmessvorrichtung (112), die an der Einspritzleitung (111) angeordnet ist, um eine oder mehrere Durchflussmessungen des in der Einspritzleitung (111) strömenden NO-haltigen Gases durchzuführen,
- einen Notkreislauf (200) mit einer Notleitung (201), die stromaufwärts (111a) und stromabwärts (111b) der Ventilvorrichtung (113) fluidisch mit der Einspritzleitung (111) verbunden ist, wobei die Notleitung (201) ein Notmagnetventil (202), das so konfiguriert ist, dass es sich normalerweise in einer offenen Position befindet, um einen Gasfluss in der Notleitung (201) zu ermöglichen, und eine Durchflusssteuervorrichtung (210) umfasst, und
- Steuermittel (130), die so konfiguriert sind, dass sie mit dem Notmagnetventil (202), der Durchflussregelvorrichtung (210), der Ventilvorrichtung (113) und der Durchflussmessvorrichtung (112) zusammenwirken,
und wobei im Falle einer Fehlfunktion, die zu einem Ausfall der Zusammenarbeit mit den Steuermitteln (130) führt:
- das Notmagnetventil (202) so konfiguriert ist, dass es in die geöffnete Position wechselt, um einen Gasfluss in der Notleitung (201) des Notkreislaufs (200) zu ermöglichen,
- die Ventilvorrichtung (113) so konfiguriert ist, dass sie in die geschlossene Position wechselt, um jeglichen Gasfluss in der Einspritzleitung (111) zu stoppen, und
- die Durchflussregelvorrichtung (210) so konfiguriert ist, dass sie das Gas mit einem voreingestellten Notgasdurchfluss liefert, wobei der Notgasdurchfluss
∘ von der Steuereinrichtung (130) anhand mindestens einer von der Durchflussmesseinrichtung (112) während des normalen Betriebs des NO-Abgabevorrichtungs (1) vor der genannten Fehlfunktion gelieferten Gasdurchflussmessung bestimmt wird und
∘ durch Steuerung der Durchflusssteuervorrichtung (210) durch die Steuermittel (130) während des normalen Betriebs des NO-Abgabevorrichtungs (1) voreingestellt wird,
**dadurch gekennzeichnet, dass**:
- ein Mehrwegeventil (205) in der Notleitung (201) stromabwärts der Durchflusssteuervorrichtung (210) angeordnet ist,
- wobei das Mehrwegeventil (205) umfasst:
∘ einen Einlasskanal, der strömungstechnisch mit der Notleitung (201) stromabwärts der Durchflussregelvorrichtung (210) verbunden ist,
∘ einen ersten Auslasskanal, der fluidisch mit einer ersten Dosierleitung (206) verbunden ist, die eine erste Kalibrierungsvorrichtung (208) umfasst, und
∘ einen zweiten Auslasskanal, der fluidisch mit einer zweiten Dosierleitung (207) verbunden ist, die eine zweite kalibrierte Öffnungsvorrichtung (209) umfasst,
- wobei die erste Dosierleitung (206) und die zweite Dosierleitung (207) stromabwärts der ersten und zweiten Kalibrierungsvorrichtung (208, 209) an die Notleitung (201) angeschlossen sind (201b),
- die erste und die zweite Kalibrierungsvorrichtung (208, 209) Kalibrierungsöffnungen (D1, D2) mit unterschiedlichen Durchflussquerschnitten oder Durchmessern aufweisen,
- und die Steuermittel (130) sind so konfiguriert, dass sie das Mehrwegeventil (205) steuern, um den Gasstrom entweder zur ersten Dosierleitung (206) oder alternativ zur zweiten Dosierleitung (207) zu leiten, und zwar in Abhängigkeit von der oder den Gasdurchflussmessungen, die von der Durchflussmesseinrichtung (112) während des normalen Betriebs der Vorrichtung (1) vor der genannten Fehlfunktion geliefert wurden.

2. Vorrichtung nach Anspruch 1, **dadurch gekennzeichnet, dass** das Mehrwegeventil (205) drei Wege umfasst.

3. Vorrichtung nach Anspruch 1, **dadurch gekennzeichnet, dass** die erste kalibrierte Öffnung der ersten kalibrierten Öffnungsvorrichtung (208) einen ersten Durchgangsdurchmesser (D1) und die kalibrierte Öffnung der zweiten kalibrierten Öffnungsvorrichtung (209) einen zweiten Durchgangsdurchmesser (D2) aufweist, sodass 1,5.D1 < D2 < 4.D1 gilt.

4. Vorrichtung nach Anspruch 3, **dadurch gekennzeichnet, dass** der erste Durchgangsdurchmesser (D1) und der zweite Durchgangsdurchmesser (D2) wie folgt sind:
1,8.D1 < D2 < 3.D1, vorzugsweise ist D2 gleich etwa 2.D1.

5. Vorrichtung nach Anspruch 1, **dadurch gekennzeichnet, dass** das Notmagnetventil (202) vom Typ "alles oder nichts" ist, das nur einen offenen Zustand, in dem es den Gasstrom durchlässt, und einen geschlossenen Zustand, in dem es den Durchfluss des Gasstroms unterbricht, annehmen kann.

6. Vorrichtung nach Anspruch 1, **dadurch gekennzeichnet, dass** die Durchflusssteuerungseinrichtung (210) ein Betätigungsmittel (203) umfasst, das mit einem pneumatischen Druckregler (204) zusammenwirkt, wobei das Betätigungsmittel (203) vorzugsweise einen Schrittmotor umfasst.

7. Vorrichtung nach Anspruch 6, **dadurch gekennzeichnet, dass**:
- der pneumatische Regler (204) so konfiguriert ist, dass er auf mehrere Druckstufen zwischen 0 und 2 bar relativ, vorzugsweise weniger als 1,5 bar relativ, einstellbar ist, und
- das Betätigungsmittel (203) mit dem pneumatischen Regler (204) zusammenwirkt, um einen gewünschten Ausgangsdruck stromabwärts des pneumatischen Reglers (204) festzulegen.

8. Vorrichtung nach Anspruch 7, **dadurch gekennzeichnet, dass**:
- der pneumatische Regler (204) eine interne Feder umfasst, die es ermöglicht, den gewünschten Druckpegel einzustellen, und
- die Betätigungseinrichtung (203) einen Schrittmotor umfasst, der so konfiguriert ist, dass er mehrere verschiedene Winkelpositionen einnehmen kann, wobei jede Winkelposition des Schrittmotors einer bestimmten Spannung der internen Feder des pneumatischen Reglers (204) entspricht,
sodass das gewünschte Ausgangsdruckniveau stromabwärts des pneumatischen Reglers (204) durch die Spannung der internen Feder des pneumatischen Reglers (204) bestimmt wird, die der vom Schrittmotor eingenommenen Winkelposition entspricht.

9. Vorrichtung nach Anspruch 1, **dadurch gekennzeichnet, dass** im Normalbetrieb die Steuermittel (130) so konfiguriert sind, dass sie das Mehrwegeventil (205) so steuern, dass eine Fluidverbindung zwischen dem Einlasskanal des Mehrwegeventils (205) und entweder dem ersten oder dem zweiten Ausgang des Mehrwegeventils (205) herzustellen, um den Gasstrom durch die erste oder die zweite Kalibrierungsöffnungseinrichtung (208, 209) zu leiten.

10. Vorrichtung nach Anspruch 1, **dadurch gekennzeichnet, dass** die Steuereinrichtung (130) mindestens einen Mikroprozessor umfasst.

11. Vorrichtung nach Anspruch 1, **dadurch gekennzeichnet, dass** die Ventilanordnung (113) ein Proportionalmagnetventil umfasst.

12. Vorrichtung nach Anspruch 1, **dadurch gekennzeichnet, dass** die Durchflussmessvorrichtung (112) so konfiguriert ist, dass sie während des normalen Betriebs der NO-Abgabevorrichtung (1) mehrere aufeinanderfolgende Durchflussmessungen durchführt.

13. Vorrichtung nach den Ansprüchen 1 und 6, 7 oder 8, **dadurch gekennzeichnet, dass** die Steuermittel (130) so konfiguriert sind, dass sie die Öffnung des pneumatischen Druckreglers (204) und/oder den Notdurchfluss anhand einer durch Speichermittel, wie beispielsweise einen Computerspeicher, gespeicherten Zuordnungstabelle bestimmen.

14. Vorrichtung zur Versorgung eines Patienten mit Gas (1, 2), umfassend:
- mindestens eine NO-Quelle (250), die ein NO/N₂-Gasgemisch enthält, vorzugsweise ein NO/N₂-Gasgemisch, das zwischen 100 und 2000 ppmv NO enthält, wobei der Rest Stickstoff (N₂) ist,
- eine NO-Abgabevorrichtung (1) gemäß einem der vorstehenden Ansprüche, die von der mindestens einen NO-Quelle (250) mit dem NO/N₂-Gasgemisch versorgt wird,
- ein Inspirationszweig (31) eines Patientenkreislaufs (3), der mit einem NO/N₂-Gasgemisch durch das NO-Abgabevorrichtung (1) versorgt wird, und
- ein medizinisches Beatmungsgerät (2) in Fluidverbindung mit dem Inspirationszweig (31), um den Inspirationszweig (31) mit einem Atemgas zu versorgen, das mindestens 20 % Sauerstoff enthält, vorzugsweise Luft oder ein Sauerstoff/Stickstoff-Gemisch.

## Claims

1. NO delivery device (1) for supplying a gas containing NO, in particular an NO/N₂ gas mixture, comprising:
- an NO injection line (111) for conveying the NO-containing gas,
- a valve device (113) arranged on the injection line (111) to control the flow of the NO-containing gas in the injection line (111), said valve device (113) being configured to be normally in a closed position to prevent any gas flow in the injection line (111),
- a flow measurement device (112) arranged on the injection line (111) to measure the flow rate of the NO-containing gas flowing in the injection line (111),
- a backup circuit (200) comprising a backup line (201) fluidly connected to the injection line (111), upstream (111a) and downstream (111b) of the valve device (113), said backup line (201) comprising a backup solenoid valve (202) configured to be normally in an open position to allow gas to flow through the backup line (201), and a flow control device (210), and
- control means (130) configured to cooperate with the emergency solenoid valve (202), the flow control device (210), the valve device (113) and the flow measurement device (112),
and wherein, in the event of a malfunction causing a failure of cooperation with the control means (130):
- the emergency solenoid valve (202) is configured to switch to the open position to allow gas to flow through the emergency line (201) of the emergency circuit (200),
- the valve device (113) is configured to switch to the closed position to stop all gas flow in the injection line (111), and
- the flow control device (210) is configured to supply gas at a preset emergency gas flow rate, wherein said emergency gas flow rate:
∘ is determined by the control means (130) from at least one gas flow measurement provided by the flow measurement device (112) during normal operation of the device (1) preceding said malfunction, and
∘ is preset by control of said flow control device (210) by the control means (130) during said normal operation of the device (1),
**characterized in that**:
- a multi-way solenoid valve (205) is arranged on the backup line (201), downstream of the flow control device (210),
- said multi-way solenoid valve (205) comprising:
∘ an inlet port fluidically connected to the backup line (201) downstream of the flow control device (210),
∘ a first outlet port fluidly connected to a first metering line (206) comprising a first calibrated orifice device (208), and
∘ a second outlet port fluidly connected to a second metering line (207) comprising a second calibrated orifice device (209),
- the first metering line (206) and the second metering line (207) connect (201b) to the backup line (201) downstream of said first and second calibrated orifice devices (208, 209),
- the first and second calibrated orifice devices (208, 209) have calibrated orifices (D1, D2) with different passage cross-sections or diameters,
- and the control means (130) are configured to control the multi-way solenoid valve (205) to direct the gas flow to the first metering line (206) or, alternatively, to the second dosing line (207) depending on the gas flow measurement(s) provided by the flow measurement device (112) during normal operation of the device (1) preceding said malfunction.

2. Device according to claim 1, **characterized in that** the multi-way solenoid valve (205) comprises 3 ways.

3. Device according to claim 1, **characterized in that** the first calibrated orifice of the first calibrated orifice device (208) has a first passage diameter (D1) and the calibrated orifice of the second calibrated orifice device (209) has a second passage diameter (D2) such that 1.5.D1 < D2 < 4.D1.

4. Device according to claim 3, **characterized in that** the first passage diameter (D1) and the second passage diameter (D2) are such that:
1.8.D1 < D2 < 3.D1, preferably D2 is equal to approximately 2.D1.

5. Device according to claim 1, **characterized in that** the backup solenoid valve (202) is of the on/off type that can only adopt an open state in which it allows the gas flow to pass and a closed state in which it interrupts the passage of the gas flow.

6. Device according to claim 1, **characterized in that** the flow control device (210) comprising an actuator means (203) cooperating with a pneumatic pressure regulator (204), preferably the actuator means (203) comprises a stepper motor.

7. Device according to claim 6, **characterized in that**:
- the pneumatic regulator (204) is configured to be adjustable to several pressure levels between 0 and 2 bar relative, preferably less than 1.5 bar relative, and
- the actuator means (203) cooperates with the pneumatic regulator (204) to set a desired outlet pressure downstream of said pneumatic regulator (204).

8. Device according to claim 7, **characterized in that**:
- the pneumatic regulator (204) comprises an internal spring for setting the desired pressure level, and
- the actuator means (203) comprises a stepper motor configured to adopt several different angular positions, each angular position of the stepper motor corresponding to a given tension of the internal spring of the pneumatic regulator (204),
so that the desired outlet pressure level downstream of the pneumatic regulator (204) is determined by the tension of the internal spring of the pneumatic regulator (204) corresponding to the angular position adopted by the stepper motor.

9. Device according to claim 1, **characterized in that**, in normal operation, the control means (130) are configured to control the multi-way solenoid valve (205) to operate a fluidic communication between the inlet port of the multi-way solenoid valve (205) and either the first or second outlet port of the multi-port solenoid valve (205) so as to pass the gas flow through the first or second calibrated orifice device (208, 209).

10. Device according to claim 1, **characterized in that** the control means (130) comprise at least one microprocessor.

11. Device according to claim 1, **characterized in that** the valve device (113) comprises a proportional solenoid valve.

12. Device according to claim 1, **characterized in that** the flow measurement device (112) is configured to perform several successive flow measurements during normal operation of the NO delivery device (1).

13. Device according to claims 1 and 6, 7 or 8, **characterized in that** the control means (130) are configured to determine the opening of the pneumatic pressure regulator (204) and/or the backup flow rate from a correspondence table stored by storage means, such as a computer memory.

14. Gas supply system (1, 2) for a patient, comprising:
- at least one NO source (250) containing an NO/N₂gas mixture, preferably an NO/N₂gas mixture containing between 100 and 2000 ppmv of NO, the remainder being nitrogen (N₂),
- an NO delivery device (1) according to one of the preceding claims, supplied with NO/N₂ gas mixture by said at least one NO source (250),
- an inspiratory branch (31) of a patient circuit (3) supplied with a NO/N₂ gas mixture by the NO delivery device (1) , and
- a medical ventilator (2) in fluid communication with the inspiratory branch (31) to supply said inspiratory branch (31) with a respiratory gas containing at least 20% oxygen, preferably air or an oxygen/nitrogen mixture.
